# EUROPEAN PATENT SPECIFICATION

(11) **EP 3 936 071 B1**
(45) Date of publication and mention of the grant of the patent: **10.04.2024**
(21) Application number: 21194171.1
(22) Date of filing: 23.10.2019
(51) Int. Cl.: A61B 18/18, A61B 18/14, A61B 17/00

(54) **HANDPIECE FOR AN ELECTROSURGICAL INSTRUMENT**
HANDSTÜCK FÜR EIN ELEKTROCHIRURGISCHES INSTRUMENT
PIÈCE À MAIN POUR INSTRUMENT ÉLECTROCHIRURGICAL

(30) Priority: 30.10.2018 GB 201817703
(43) Date of publication of application: 12.01.2022
(62) Divisional of application: 19795158.5
(73) Proprietor: Creo Medical Limited, Chepstow, Monmouthshire NP16 5UH (GB)
(72) Inventor: HANCOCK, Christopher Paul, Bath (GB); ULLRICH, George, Bangor (GB); PRESTON, Shaun, Chepstow (GB); WEBB, David, Bangor (GB); SWAIN, Sandra, Chepstow (GB); WHITE, Malcolm, Chepstow (GB)
(74) Representative: Mewburn Ellis LLP

(56) References cited:
- EP-A1- 2 996 768
- US-A1- 2016 361 088
- US-A1- 2017 231 695

## Description

### FIELD OF THE INVENTION

The invention relates to a handpiece for controlling movement of an electrosurgical device along a lumen of a catheter. The invention further relates to an electrosurgical system for delivering electromagnetic energy to biological tissue in order to ablate target tissue. In particular, the probe is configured to be insertable through a channel of a surgical scoping device or catheter that can be introduced to a treatment site in a non-invasive manner. The probe may be arranged to ablate tissue, such as a tumour, cyst or other lesion. The probe may be particularly suited for treatment in the pancreas.

### BACKGROUND TO THE INVENTION

Electromagnetic (EM) energy, and in particular microwave and radiofrequency (RF) energy, has been found to be useful in electrosurgical operations, for its ability to cut, coagulate, and ablate body tissue. Typically, apparatus for delivering EM energy to body tissue includes a generator comprising a source of EM energy, and an electrosurgical instrument connected to the generator, for delivering the energy to tissue. Conventional electrosurgical instruments are often designed to be inserted percutaneously into the patient's body. However, it can be difficult to locate the instrument percutaneously in the body, for example if the target site is in a moving lung or a thin walled section of the gastrointestinal (GI) tract. Other electrosurgical instruments can be delivered to a target site by a surgical scoping device (e.g. an endoscope) which can be run through channels in the body such as airways or the lumen of the oesophagus or colon. This allows for minimally invasive treatments, which can reduce the mortality rate of patients and reduce intraoperative and postoperative complication rates.

A technique of treating tissue in the pancreas using endoscopic ultrasound guided radiofrequency ablation is known (Pai, M., et al.: Endoscopic ultrasound guided radiofrequency ablation, for pancreatic cystic neoplasms and neuroendocrine tumors, World J Gastrointest Surg 2015 April 27; 7(4): 52-59). In this technique a conductive wire having a small diameter (e.g. 0.33 mm) is inserted through the working channel of an ultrasound-enabled endoscope. RF power is applied to the wire in conjunction with an external grounded return pad in contact with the patient's skin to coagulate tissue in the liver and pancreas. To ablate lesions it is necessary to apply power for 90-120 seconds, and, in some cases to remove and reposition the wire.

US 2016/361088 A1 discloses a system including a handle, a cannula removably disposed within a lumen of the handle, and a penetration member sized to advance through a lumen of the cannula, the cannula configured to passively transition to a nonlinear shape as the penetration member is withdrawn from a distal section of the cannula. A cannula hub is configured to selectively engage with a proximal end of the handle to selectively lock rotational movement of the cannula with respect to the handle.

### SUMMARY OF THE INVENTION

The invention is defined by independent claim 1.

Preferred embodiments are defined in the dependent claims.

At its most general, the invention provides an electrosurgical device having a radiating tip portion for delivering electromagnetic energy to biological tissue, where the electrosurgical device is disposed in a catheter. The electrosurgical device is movable relative to the catheter between a deployed position where the radiating tip portion is exposed and a retracted position where the radiating tip portion is contained within the catheter. In this manner, the radiating tip portion may be retracted until the moment when it is to be used. This may facilitate insertion of the device through an instrument channel of a surgical scoping device. In particular, this may prevent the radiating tip portion from catching on the instrument channel when the device is inserted into the instrument channel, which could cause damage to the instrument channel and/or radiating tip portion.

This configuration may be particularly beneficial for treatment of tumours in the pancreas, as the device may be positioned adjacent to the duodenum wall with the radiating tip portion in the retracted position. Then, the radiating tip portion may be exposed to pierce the duodenum wall and penetrate the pancreas, where it may deliver electromagnetic energy to ablate target tissue. The radiating tip portion may be dimensioned to be suitable for insertion into a pancreas, to provide a rapid and accurate alternative to known RF ablation techniques.

Although the invention may find particular use in the pancreas, it may also be suitable for use in other awkward treatment sites, such as the lungs, liver, etc.

According to a first aspect of the invention, there is provided an electrosurgical instrument comprising: a flexible catheter having a lumen extending therethrough, the catheter being dimensioned to be insertable through an instrument channel of a surgical scoping device; and an electrosurgical device disposed within the lumen, the electrosurgical device comprising: a flexible coaxial cable configured to convey microwave energy; and a radiating tip portion connected at a distal end of the coaxial cable to receive the microwave energy, the radiating tip portion having a smaller outer diameter than the flexible coaxial cable, wherein the radiating tip portion comprises: a proximal coaxial transmission line for conveying the microwave energy; and a distal needle tip at a distal end of the proximal coaxial transmission line, the distal needle tip being configured to radiate the microwave energy into biological tissue, wherein the electrosurgical device is longitudinally movable within the lumen between a deployed position in which the distal needle tip protrudes beyond a distal end of the catheter, and a retracted position in which the distal needle tip portion is contained within the catheter.

This configuration enables the radiating tip portion to be retracted into the catheter when it is not in use. In this manner, the instrument may be manoeuvred into position with the radiating tip portion in the retracted position, in order to facilitate manoeuvring of the instrument. As the radiating tip portion has a smaller outer diameter than the coaxial cable, it may be more likely to catch in the instrument channel of a surgical scoping device, or on tissue. By keeping the radiating tip portion in the retracted position while the instrument is being manoeuvred, such catching of the radiating tip portion can be avoided.

The electrosurgical device may be moved along the lumen of the catheter using a suitable actuation mechanism. When the electrosurgical device is in the deployed position, all or a portion of the radiating tip portion may protrude beyond the distal end of the catheter. When the electrosurgical device is in the retracted position, the distal needle tip may be located inside the catheter so that it does not protrude beyond the distal end of the catheter. The electrosurgical device may be moved in a distal direction to move it into the deployed position, and the electrosurgical instrument may be moved in a proximal direction to move it into the retracted position.

The catheter may be formed by a tube made of a flexible bio-compatible material. For example, the catheter may be made of a tube of polyether ether ketone (PEEK) or PTFE. As another example, the catheter may be made of a polyether block amide (e.g. Pebax^{®}) material. The catheter may be made of, or coated with, a non-stick material (e.g. PTFE) to prevent tissue from sticking to the catheter. To fit within the instrument channel of a surgical scoping device, the catheter may have an outer diameter equal to or less than 2.0 mm.

The lumen may be a longitudinal passageway extending through the catheter. The lumen may be dimensioned to receive and permit passage of the electrosurgical device.

The flexible coaxial cable may be a conventional low loss coaxial cable that is connectable at a proximal end to an electrosurgical generator, to receive the microwave energy. The coaxial cable may have a centre conductor separated from an outer conductor by a dielectric material. The coaxial cable may further include an outer protective sheath for insulating and protecting the cable. In some examples, the protective sheath may be made of or coated with a non-stick material to facilitate movement of the coaxial cable along the lumen. The radiating tip portion is located at the distal end of the coaxial cable, and is connected to receive the microwave energy conveyed along the coaxial cable.

The proximal coaxial transmission line is connected to the distal end of coaxial cable, to receive the microwave energy conveyed by the coaxial cable. The proximal coaxial transmission line may have an inner conductor that is electrically connected to the centre conductor of the coaxial cable. The proximal coaxial transmission line may further have a proximal dielectric sleeve disposed around the inner conductor, and an outer conductor formed around the proximal dielectric sleeve. The outer conductor of the proximal coaxial transmission line may be electrically connected to the outer conductor of the coaxial cable.

The materials used in the proximal coaxial transmission line may be the same or different to those used in the coaxial cable. The materials used in the proximal coaxial transmission line may be selected to provide a desired flexibility and/or impedance of the proximal coaxial transmission line. For example, the dielectric material of the proximal coaxial transmission line may be selected to improve impedance matching with target tissue.

The dimensions of the components of the proximal coaxial transmission line may be chosen to provide it with an impedance that is identical or close to the impedance of the flexible coaxial cable (e.g. around 50 Q). The inner conductor may be formed from a material with high conductivity, e.g. silver or copper. The inner conductor of the proximal coaxial transmission line may have a smaller outer diameter than the centre conductor of the coaxial cable. This may facilitate bending of the radiating tip portion.

The distal needle tip is formed at the distal end of the proximal coaxial transmission line. The distal needle tip may include an emitter structure which is arranged to receive the microwave energy from the proximal coaxial transmission line and deliver the energy into target tissue. The emitter structure may be selected based on the type of energy to be delivered, and a desired treatment modality. For example, the emitter structure may include a monopolar or bipolar microwave antenna for radiating microwave energy into surrounding tissue to perform tissue ablation. In other examples, the emitter structure may include a pair of RF electrodes which are arranged to deliver radiofrequency energy into target tissue. In such examples, the coaxial cable is arranged to deliver radiofrequency (RF) energy to the electrosurgical device, which may be capable of performing ablation, coagulation and/or resection using the RF energy. In some examples, the emitter structure may be configured to emit both microwave and radiofrequency energy (either simultaneously or sequentially).

As mentioned above, the outer diameter of the radiating tip portion is smaller than the outer diameter of the coaxial cable. By using a smaller diameter radiating tip portion, the size of an insertion hole produced when inserting the radiating tip portion into target tissue may be reduced. This may reduce bleeding, and enable faster healing of the wound. For example, this may reduce the size of an insertion hole made in the duodenum wall when the device is inserted into the pancreas. The inventors have also found that such a configuration is beneficial for treatment of tumours in the liver, where bleeding of an insertion hole may be an issue.

Additionally, by making the outer diameter of the radiating tip portion smaller than the coaxial cable, the radiating tip portion may be more flexible than the coaxial cable. This may facilitate guiding the distal needle tip to a desired location, e.g. where it is necessary to guide the device around a tight bend. The invention thus strikes a balance between using a coaxial cable with a relatively large outer diameter to reduce thermal loss effects and a radiating tip portion with a relatively small diameter to provide improved flexibility and reduce tissue damage on insertion.

The distal needle tip may be configured to operate as a half wavelength transformer to deliver the microwave energy from the distal needle tip into biological tissue. An advantage of configuring the distal needle tip as a half wavelength transformer may be to minimise reflections at the interface between components, e.g. between the coaxial cable and proximal coaxial transmission line, and between the proximal coaxial transmission line and the distal needle tip. A reflection coefficient at the latter interface is typically larger due to a larger variation in impedance. The half wavelength configuration may minimise these reflections so that the dominant reflection coefficient becomes that of the interface between the proximal coaxial transmission line and the tissue. The impedance of the proximal coaxial transmission line may be selected to be identical or close to the expected tissue impedance to provides a good match at the frequency of the microwave energy.

The catheter may include a constricted passageway at its distal end, the constricted passageway being dimensioned to permit passage of the radiating tip portion and to prohibit passage of the flexible coaxial cable. In an example, the constricted passageway may extend through a plug mounted at the distal end of the catheter. The plug may act to prevent tissue and/or fluid from entering the catheter from a treatment site. Moreover, the constricted passageway may serve to guide the radiating tip portion as the device is moved between the retracted position and the deployed position. This may enable accurate positioning of the radiating tip portion, which may facilitate guiding the distal needle tip to a target treatment site. The constricted passageway may be aligned with a longitudinal axis of the instrument, to centralise the radiating tip portion about the longitudinal axis. The plug may be made of an insulating material (e.g. PEEK), to avoid shorting between the plug and the radiating tip portion.

The plug may cover an opening at the distal end of the catheter, e.g. it may be a cap on the distal end of the catheter. The constricted passageway may be dimensioned to allow the radiating tip portion to pass through it, but to prevent passage of the larger diameter coaxial cable. In this manner, the constricted passageway may act to limit movement of the electrosurgical device along the lumen in a distal direction. This may prevent the coaxial cable from being pushed beyond the distal end of the catheter, which may prevent injury due to the larger diameter of the coaxial cable.

In some embodiments, the constricted passageway may include a lip for removing biological tissue from the radiating tip portion when the electrosurgical device is moved from the deployed position to the retracted position. For example, the lip may be arranged to scrape tissue off the radiating tip portion when the radiating tip portion is retracted into the catheter. This may prevent tissue from being entrained inside the catheter, which could contaminate the catheter or cause malfunction of the instrument (e.g. by restricting motion of the electrosurgical device along the lumen). The lip may be disposed at the distal opening of the passageway, e.g. the lip may be disposed around the distal opening of the passageway. In this manner, tissue may be prevented from entering the passageway. The lip may be a sharp edge around the opening of the passageway.

A distal surface of the catheter may be rounded. For example, the distal end face of the plug may be rounded, e.g. have a hemispherical or domed form. The rounded surface may avoid the presence of any sharp edges around the distal end of the catheter. This may facilitate inserting the instrument down a working channel of a surgical scoping instrument. In particular, it may facilitate moving the instrument through a bend in the working channel.

The distal needle tip may be retained in the constricted passageway when in the retracted position. For example, a proximal opening of the constricted passageway may be located inside the catheter, and a distance between the proximal opening of the constricted passageway and the distal opening of the constricted passageway may be greater than a length of the distal needle tip. In other words, a length of the constricted passageway may be greater than the length of the distal needle tip. In this manner, the distal needle tip may be fully contained within the constricted passageway when the radiating tip portion is in the retracted position. The constricted passageway may be defined in a body portion of the plug that is contained within a distal section of the catheter.

The proximal coaxial transmission line may have a larger outer diameter than the distal needle tip, e.g. due to the outer conductor of the proximal coaxial transmission line. As a result, there may be a lip or a step at an interface between the distal needle tip and the proximal coaxial transmission line. By making the constricted passageway longer than the distal needle tip, the lip at the interface between the distal needle tip and the proximal coaxial transmission line may be located inside the constricted passageway when the device is in the retracted position. In this manner, catching of the lip on the proximal opening of the constricted passageway is prevented when the device is moved from the retracted position to the deployed position.

The proximal opening of the passageway in the plug may be flared outwards, e.g. a diameter of the proximal opening may increase in a proximal direction. In this manner, the proximal opening of the passageway may act as a funnel that guides the radiating tip portion into the passageway. This may facilitate moving the electrosurgical device along the lumen, and may avoid the radiating tip portion from catching on the proximal opening of the passageway.

In some embodiments, the plug may include a body portion disposed inside the catheter, the body portion comprising a protrusion for securing the plug to the catheter. In this manner, the plug may be mounted on the distal end of the catheter without use of adhesive. In some cases however, adhesive may be used to further secure the plug to the catheter. The protrusion may, for example, be a bulge or a barb arranged to press outwards against the catheter to hold the plug in place in the catheter. The plug may be mounted on the distal end of the catheter by pushing the body portion of the plug into the catheter. In this manner, a "push-fit" connection may be formed between the plug and the distal end of the catheter.

The distal needle tip may comprise a pointed tip at its distal end. The pointed tip may be made of a rigid insulating material, such as zirconia or a ceramic. Zirconia is a rigid dielectric material which may be sharpened to a fine point, and so may be particularly suitable for use as the pointed tip. The pointed tip may serve to pierce tissue, to facilitate insertion of the radiating tip portion into target tissue.

The distal needle tip may comprise a distal dielectric sleeve around a central conductive element, and the pointed tip may be secured in a bore at a distal end of the distal dielectric sleeve. The pointed tip may include a body which is disposed at the distal end of the distal dielectric sleeve, wherein the body of the pointed tip may include a protrusion for securing the body in the bore. In this manner, the pointed tip may be held in the bore at the distal end of the distal dielectric sleeve. By providing the body of the pointed tip in the bore in the distal dielectric sleeve, the pointed tip may be securely held in place. The protrusion may, for example, be a bulge or a barb arranged to press outwards against a wall of the bore to hold the body in place in the bore. The pointed tip may be mounted on the distal end of the distal dielectric sleeve by pushing the body of the distal tip into the bore. In this manner, a "push-fit" connection may be formed between the pointed tip and the distal dielectric sleeve. This configuration may enable the pointed tip to be mounted on the distal dielectric sleeve without adhesive. In some cases however, adhesive may be used to further secure the pointed tip in place.

The pointed tip may be made of a dielectric material having a higher rigidity than the distal dielectric sleeve. This may enable the pointed tip to be sharper, to facilitate piercing of tissue.

The proximal coaxial transmission line may comprise an inner conductor separated from an outer conductor by a dielectric sleeve, wherein the inner conductor comprises a distal portion that protrudes beyond a distal end of the outer conductor, and wherein the distal needle tip comprises a length of the distal portion of the inner conductor. The distal dielectric sleeve may thus be around the inner conductor, which forms the central conductive element mentioned above.

The distal dielectric sleeve may be made from the same or a different material compared to the dielectric material in the proximal coaxial transmission line. The distal dielectric sleeve may have a higher rigidity than the dielectric material of the proximal coaxial transmission line. Providing a higher rigidity to the distal dielectric sleeve may facilitate insertion of the distal needle tip into target tissue, whilst having a lower rigidity proximal coaxial transmission line may facilitate bending of the radiating tip portion. This may enable the instrument to be guided through narrow and winding passageways, whilst still enabling it to be inserted into target tissue. For example, the dielectric material of the proximal coaxial transmission line may be made of a flexible dielectric material (e.g. PTFE), and the distal dielectric sleeve may be made of e.g. a ceramic, polyether ether ketone (PEEK) or glass-filled PEEK.

In some embodiments the pointed tip may have a proximal portion which is tapered at a first tapering angle relative to a longitudinal direction and a distal section which is tapered at a second tapering angle relative to the longitudinal direction, the first tapering angle being smaller than the second tapering angle. By having a larger tapering angle in the distal section of the pointed tip, fragility of the pointed tip may be reduced. The may reduce the chance of particulates breaking off from the pointed tip and remaining in the body.

The outer conductor of the proximal coaxial transmission line may be made of nitinol. For example, the outer conductor may be formed of a nitinol tube. The inventors have found that nitinol exhibits a longitudinal rigidity sufficient to transmit a force capable of penetrating the duodenum wall. Additionally, the flexibility of nitinol may facilitate bending of the radiating tip portion, so that the instrument may be guided through narrow bending passageways. Forming the outer conductor of nitinol may thus facilitate use of the instrument for treatment of tumours in the pancreas.

The radiating tip portion may be secured to the coaxial cable by a collar mounted over a junction therebetween, the collar having a distal surface that is rounded. The collar may be located at an interface between the distal end of the coaxial cable and a proximal end of the proximal coaxial transmission line. The distal surface of the collar may be a forward-facing surface of the collar, i.e. a surface that faces towards the distal end of the instrument. By providing a collar with a rounded distal surface, sharp edges may be avoided at the interface between the coaxial cable and the proximal coaxial transmission line. This may reduce friction between the electrosurgical device and the catheter when the electrosurgical device is moved along the lumen. This may, for example, facilitate moving the electrosurgical device along the lumen when the instrument is in retroflex (e.g. when there is a bend in the catheter).

In some embodiments, the collar may be conductive and may electrically connect an outer conductor of the proximal coaxial transmission line to an outer conductor of the coaxial cable, and a dielectric spacer may be mounted at the junction between the radiating tip portion and the coaxial cable, the dielectric spacer being disposed between an inner conductor of the proximal coaxial transmission line and the collar. For example, the dielectric spacer may be an insulating washer mounted at the interface and disposed around a proximal end of the inner conductor. This may reduce the risk of a short occurring at the interface between the coaxial cable and the radiating tip portion and improve electrical safety of the junction.

A length of the radiating tip portion may be equal to or greater than 140 mm. Coaxial cables which are typically used in electrosurgical instruments (e.g. the Sucoform 86 coaxial cable) often have a heavily tinned outer jacket to enable longitudinal actuation of the cable. However, this results in the coaxial cable being relatively stiff, such that it requires a large force to bend the coaxial cable. This may cause a large amount of friction when the device is moved through a bend in the catheter, which may impede accurate control of the device. Having a long radiating tip portion may facilitate bending of the instrument near its distal end, as the radiating tip portion may have a greater flexibility compared to the coaxial cable. By making the radiating tip portion 140 mm or longer, it may be possible to avoid having to move the coaxial cable through a bent distal portion of the catheter. This may, for example, facilitate deploying the radiating tip portion where a distal portion of the catheter is in retroflex. This configuration may be particularly beneficial for use in the pancreas, where it may be necessary to have a distal portion of the instrument in retroflex.

The radiating tip portion may have a non-stick material on its outer surface. For example, an outer sheath may be disposed over a proximal portion of the radiating tip portion, the outer sheath being made of or coated with a non-stick material. This may facilitate moving of the electrosurgical device along the lumen, by reducing friction between the lumen and the radiating tip portion. This configuration may be particularly beneficial when combined with a long radiating tip portion (e.g. 140 mm or longer), as it may facilitate moving the radiating tip portion through a distal portion of the catheter which is in retroflex. The outer sheath may also serve to increase an effective outer diameter of the radiating tip portion, which may reduce lateral movement of the radiating tip portion in the lumen. This may improve positioning accuracy of the radiating tip portion. In some cases, the outer sheath may extend over all or a portion of a coaxial cable, to reduce friction between the coaxial cable and the lumen.

In some embodiments, the outer sheath may be made of PTFE. For example, the outer sheath may be a tube of PTFE disposed around the proximal portion of the radiating tip portion.

A proximal portion of the coaxial cable may be secured to a rigid reinforcing element. The reinforcing element may serve to increase the longitudinal rigidity of the proximal portion of the coaxial cable. This may facilitate the application of force (e.g. a pushing force or a pulling force) to the proximal end of the coaxial cable in order to move the electrosurgical device along the lumen. This may improve control of the position of the electrosurgical device in the lumen. The reinforcing element may be disposed on an outer surface of the proximal portion of the coaxial cable.

In some embodiments, the reinforcing element may be a rigid tube disposed on an outer surface of the proximal portion of the coaxial cable. The rigid tube may have a higher longitudinal rigidity than the coaxial cable. For example, the rigid tube may be a metal (e.g. stainless steel) tube.

A proximal portion of the lumen may have a larger diameter than a distal portion of the lumen, to receive the proximal portion of the coaxial cable. An outer diameter of the proximal portion of the coaxial cable may be greater than an outer diameter of a distal portion of the coaxial cable, due to the reinforcing element. Having a catheter with a larger diameter proximal portion may enable the proximal portion of the coaxial cable to slide within the catheter without additional friction caused by the reinforcing element.

In some embodiments, the catheter may include a tapered portion at its distal end, and the tapered portion may taper from a first diameter at a proximal end thereof to a second, smaller, diameter at a distal end thereof, the second diameter being larger than the outer diameter of the radiating tip portion and smaller than an outer diameter of the coaxial cable. In this manner, the tapered portion may allow passage of the radiating tip portion, so that the radiating tip portion may protrude beyond the distal end of the catheter. In contrast, the tapered portion may serve to prevent the coaxial cable (which has a larger diameter than the radiating tip portion) from being pushed through the tapered portion, which may prevent the coaxial cable from being accidentally exposed beyond the distal end of the catheter.

In some cases, the tapered portion may define a distal opening of the catheter, where a diameter of the distal opening is the second, smaller diameter. In this manner, when the electrosurgical device is in the deployed position, the radiating tip portion may protrude through the distal opening in the tapered portion. Thus, the tapered portion may serve a similar function to the plug discussed above. The tapered portion may be used as an alternative to the plug at the distal end of the catheter, e.g. to simplify construction of the instrument. In some cases, the plug may be combined with the tapered portion, e.g. the plug may be mounted in the distal opening of the tapered portion.

In some embodiments, an inner conductor of the proximal coaxial transmission line may be formed of an inner core made of a first conductive material and an outer conductive coating made of a second conductive material having a higher conductivity than the first conductive material. The first conductive material may have a higher rigidity than the second conductive material. This may increase the longitudinal rigidity of the radiating tip portion, which may facilitate transmission of a force along the radiating tip portion, e.g. for piercing tissue. For example, the inner core may be made of stainless steel, and the outer conductive coating may be made of silver.

According to a second aspect of the invention, there is provided a handpiece for controlling movement of an electrosurgical device along a lumen of a catheter, the handpiece comprising: a first section having a connector for connecting a distal end of the handpiece to an instrument port of a surgical scoping device; a second section connected to the first section and movable along a length of the first section, the second section having a holder for holding a proximal end of the catheter, whereby relative movement between the second section and first section is arranged to control a length of catheter that extends out of a distal end of the handpiece; and a third section connected to the second section and movable along a length the second section, the third section having a coaxial connector arranged to receive a proximal end of a coaxial cable that is conveyed within the lumen of the catheter, whereby relative movement between the third section and second section is arranged to control a relative position of the coaxial cable within the catheter.

The handpiece of the second aspect of the invention may be used with the electrosurgical instrument of the first aspect of the invention to control the position of the electrosurgical device in the catheter. An independent aspect of the invention may provide an electrosurgical apparatus including the electrosurgical instrument of the first aspect and the handpiece of the second aspect which is arranged to control the position of the electrosurgical device in the catheter.

Advantageously, the handpiece of the second aspect of the invention enables a length of the catheter to be adjusted independently of the position of the electrosurgical device in the catheter. In this manner, the length of the catheter may be adjusted so that it fits within the instrument channel of the surgical scoping device.

In use, the proximal end of the catheter of the electrosurgical instrument may be held in the holder of the second section. In this manner, the position of the catheter may be fixed relative to the second section. The second section may include a fixing mechanism for fixing a position of the second section relative to the first section. The fixing mechanism may for example include a clip, a clamp or some other suitable mechanism for holding the proximal end of the catheter and securing it in place.

The proximal end of the coaxial cable may be connected to the coaxial connector in the third section. In this manner, the position of the coaxial cable (and hence the electrosurgical device) may be fixed relative to the third section. For example, the coaxial cable may include a connector at its proximal end which is configured to mate with the coaxial connector in the third section. Alternatively, the coaxial cable may be connected directly to the coaxial connector in the third section, e.g. via soldered and/or welded electrical connections. The coaxial connector of the third section may be secured to a body of the third section, e.g. via screws or with an adhesive. The coaxial connector is connectable to an electrosurgical generator, e.g. via an interface cable, to receive electromagnetic energy from the generator and convey it to the coaxial cable.

The first section serves to anchor the handpiece relative to the input port of the instrument channel of the surgical scoping device. The connector on the first section may be configured to mate with a corresponding connector on the surgical scoping device. For example, the connector may be a luer connector, which may enable a leak-free connection to be formed between the handpiece and the instrument channel. The connector may include a mechanism for securing the handpiece to the surgical device, e.g. via a clamp or a threaded connection.

In use, the second section may be moved relative to the first section to adjust a length of the catheter protruding from the handpiece. As the first section may be fixed relative to instrument channel of the surgical scoping device and the catheter may be fixed relative to the second section, moving the second section relative to the first section may control the length of the catheter in the instrument channel. In this manner, length of the catheter may be adjusted so that it fits the instrument channel.

Once the length of the catheter has been adjusted, the third section may be moved relative to the second section in order to move the electrosurgical device along the lumen of the catheter. As the position of the catheter may be fixed relative to the second section and the position of the electrosurgical device may be fixed relative to the third section, moving the third section relative to the second section may cause movement of the electrosurgical device along the lumen of the catheter. In this manner, the position of the electrosurgical device in the lumen may be controlled by moving the third section relative to the second section.

As mentioned above, the second section may include a fixing mechanism for fixing a position of the second section relative to the first section. In this manner, once the length of the catheter has been adjusted to a desired length, the length of the catheter may be fixed by using the fixing mechanism to fix the position of the second section relative to the first section. The fixing mechanism may be any suitable mechanism for reversibly fixing the position of the second section relative to the first section. For example, the fixing mechanism may include a screw for fixing the second section to the first section, or a clamp for clamping the second section to the first section.

The second section may include a limiter that is movable along a length of the second section, and wherein the limiter is arranged to restrict motion of the third section relative to the second section. The position of the limiter may thus be adjusted to set a desired range of motion of the third section relative to the second section. This may serve to limit the extent to which the radiating tip portion may be exposed beyond the distal end of the catheter. This may avoid accidentally pushing the radiating tip portion out too far, which could damage healthy tissue. The limiter may, for example, have a stopping surface that is arranged to abut against the third section when the third section is moved in the distal direction, to prevent further motion of the third section in the distal direction. The limiter may be a slider that is provided on a surface of the second section. The limiter may include a fixing mechanism (e.g. screw or clamp) for fixing it at a desired location along the length of the second section.

In some embodiments, the second section may include a first set of markers arranged to indicate a maximum range of motion of the electrosurgical device along the lumen, based on a position of the limiter relative to the first set of markers. In this manner, a desired range of motion of the electrosurgical device along the lumen may be set by moving the limiter to a corresponding marker on the second section. This may facilitate setting of a desired range of motion, as it may otherwise be difficult for a user to determine the exact position of the radiating tip portion. For example, the markers may indicate a length of the radiating tip portion that protrudes from the catheter when the third section abuts against the limiter.

In some embodiments, the first section may include a second set of markers arranged to indicate a length of the catheter in the instrument channel based on a position of the second section relative to the second set of markers. In this manner, a user may adjust a length of the catheter by moving the second section to a corresponding marker on the first section. This may facilitate adjusting the length of the catheter to a desired length, as it can otherwise be difficult to determine the length of the catheter when it is in the instrument channel.

In some embodiments, the first section and the second section may telescopically arranged, the second section being slidable along the length of the first section. For example, the first and second sections may have tubular concentric bodies, one of which is arranged to slide into the other. In some cases, the second section and the third section may be telescopically arranged, the third section being slidable along the length of the second section. For example, the first and second sections may have tubular concentric bodies, one of which is arranged to slide into the other. The telescopic configuration may provide for a compact design of the handpiece.

The electrosurgical instrument and handpiece discussed above may for part of a complete electrosurgical system. For example, the electrosurgical system may comprise an electrosurgical generator arranged to supply microwave energy and/or radiofrequency energy; a surgical scoping device having a flexible insertion cord for insertion into a patient's body, wherein the flexible insertion cord has an instrument channel running along its length; an electrosurgical instrument according to the first aspect of the invention, wherein the electrosurgical instrument is dimensioned to fit within the instrument channel; and a handpiece according to the second aspect of the invention, wherein a proximal end of the catheter of the electrosurgical instrument is held in the holder, a proximal end of the coaxial cable of the electrosurgical instrument is received in the coaxial connector, and the coaxial connector is connected to the electrosurgical generator to receive the microwave energy and/or radiofrequency energy.

The term "surgical scoping device" may be used herein to mean any surgical device provided with an insertion tube that is a rigid or flexible (e.g. steerable) conduit that is introduced into a patient's body during an invasive procedure. The insertion tube may include the instrument channel and an optical channel (e.g. for transmitting light to illuminate and/or capture images of a treatment site at the distal end of the insertion tube. The instrument channel may have a diameter suitable for receiving invasive surgical tools. The diameter of the instrument channel may be 5 mm or less. In embodiments of the invention, the surgical scoping device may be an ultrasound-enabled endoscope.

Herein, the term "inner" means radially closer to the centre (e.g. axis) of the instrument channel and/or coaxial cable. The term "outer" means radially further from the centre (axis) of the instrument channel and/or coaxial cable.

The term "conductive" is used herein to mean electrically conductive, unless the context dictates otherwise.

Herein, the terms "proximal" and "distal" refer to the ends of the elongate probe. In use, the proximal end is closer to a generator for providing the RF and/or microwave energy, whereas the distal end is further from the generator.

In this specification "microwave" may be used broadly to indicate a frequency range of 400 MHz to 100 GHz, but preferably the range 1 GHz to 60 GHz. Preferred spot frequencies for microwave EM energy include: 915 MHz, 2.45 GHz, 3.3 GHz, 5.8 GHz, 10 GHz, 14.5 GHz and 24 GHz. 5.8 GHz may be preferred. The device may deliver energy at more than one of these microwave frequencies.

The term "radiofrequency" or "RF" may be used to indicate a frequency between 300 kHz and 400 MHz.

### BRIEF DESCRIPTION OF THE DRAWINGS

Embodiments of the invention are discussed below with reference to the accompanying drawings, in which:
Fig. 1 is a schematic diagram of an electrosurgical system that is an embodiment of the invention;
Figs. 2a and 2b show schematic cross-sectional views of an electrosurgical instrument that is an embodiment of the invention, where in Fig. 2a an electrosurgical device of the instrument is in a retracted position and in Fig. 2b the electrosurgical device of the instrument is in a deployed position;
Fig. 3a is a perspective view of the electrosurgical instrument of Figs. 2a and 2b;
Fig. 3b is a perspective view of the electrosurgical instrument of Figs. 2a and 2b, where a catheter of the instrument has been omitted to reveal an internal structure of the instrument;
Fig. 4 is a perspective view of the electrosurgical instrument of Figs. 2a and 2b;
Figs. 5 and 6 are schematic side views of an electrosurgical device that is a part of an electrosurgical instrument of an embodiment of the invention;
Fig. 7 shows a schematic cross-sectional view of a distal needle tip of an electrosurgical instrument that is an embodiment of the invention;
Fig. 8 shows a schematic view of a distal needle tip of an electrosurgical instrument that is an embodiment of the invention;
Figs. 9a and 9b are schematic cross-sectional views depicting deformation of respective examples of an electrosurgical instrument when inserted through an instrument channel that is in retroflex;
Fig. 10 shows a schematic cross-sectional view of a handpiece that is an embodiment of the invention;
Figs. 11 and 12 show perspective views of the handpiece of Fig. 10, where in Fig. 11 parts of components of the handpiece have been omitted to reveal an internal structure of the handpiece;
Figs. 13a and 13b show plan views of the handpiece of Fig. 10 with the distal needle in a retracted and extended (deployed) position, respectively;
Figs. 14a is a cross-sectional view of a catheter that is part of an electrosurgical instrument that is an embodiment of the invention; and
Fig. 14b is a cross-sectional view of a catheter that is part of an electrosurgical instrument that is another embodiment of the invention.

### DETAILED DESCRIPTION; FURTHER OPTIONS AND PREFERENCES

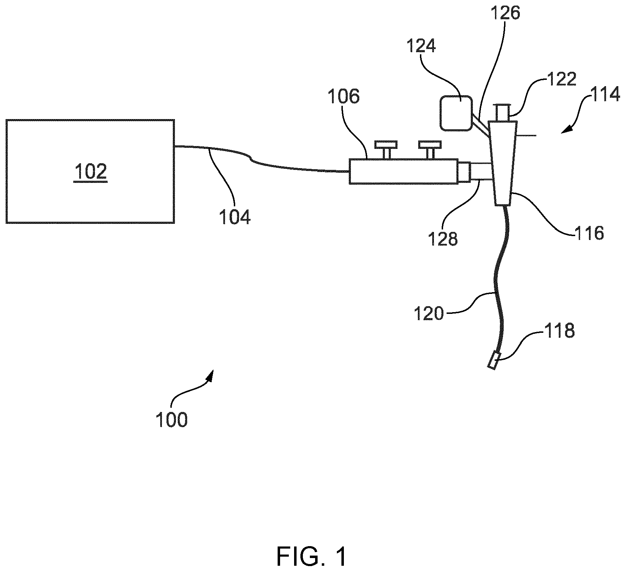

Fig. 1 is a schematic diagram of an electrosurgical ablation apparatus 100 that is capable of supplying microwave energy and/or radiofrequency energy to the distal end of an invasive electrosurgical instrument. The system 100 comprises a generator 102 for controllably supplying microwave energy and radiofrequency energy. A suitable generator for this purpose is described in WO 2012/076844.

The generator may be arranged to monitor reflected signals received back from the instrument in order to determine an appropriate power level for delivery. For example, the generator may be arranged to calculate an impedance seen at the distal end of the instrument in order to determine an optimal delivery power level.

The generator 102 is connected to a handpiece 106 by an interface cable 104. In other examples (not shown), the handpiece 106 may also be connected via a fluid flow line to a fluid delivery device, such as a syringe, e.g. where it is desired to convey fluid to the distal end of the electrosurgical instrument.

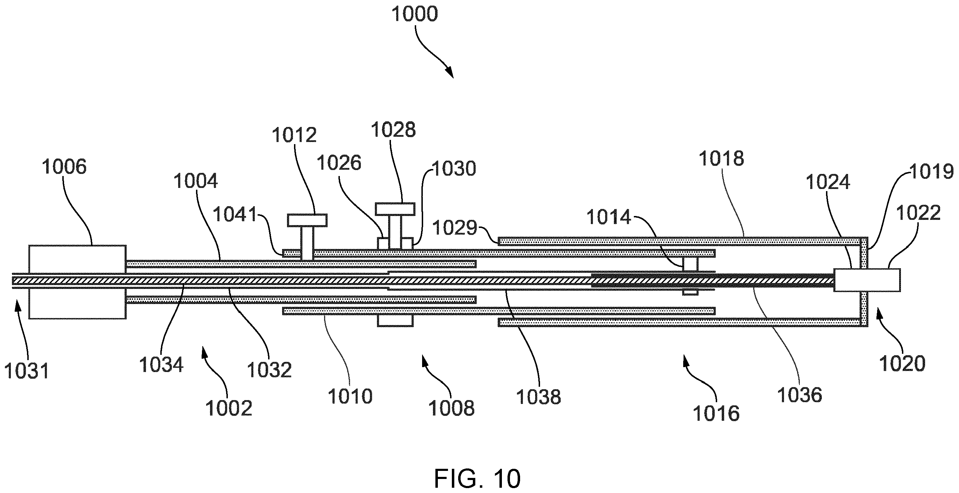

The handpiece 106 houses an instrument control mechanism that is operable to control longitudinal (back and forth) movement of the electrosurgical instrument. The handpiece 106 may also house control mechanisms (e.g. triggers) for actuating one or more control wires or push rods, if necessary. An example handpiece is described in more detail below, in relation to Figs. 10-12. A function of the handpiece is to combine the input from the generator 102 and any other inputs into an integrated flexible instrument cable which exits from a distal end of the handpiece 106 and is dimensioned to be conveyed through an instrument channel of a surgical scoping device.

The handpiece 106 is connected to an input port 128 of a surgical scoping device 114. The surgical scoping device 114 comprises a body 116 having a number of input ports and an output port from which an instrument cord 120 extends. The instrument cord 120 comprises an outer jacket which surrounds a plurality of lumens. The plurality of lumens convey various things from the body 116 to a distal end of the instrument cord 120. One of the plurality of lumens is the instrument channel through with the instrument cable extends. Other lumens may include a channel for conveying optical radiation, e.g. to provide illumination at the distal end or to gather images from the distal end, and an ultrasound signal channel for conveying an ultrasound signal. The body 116 may include an eye piece 122 or other imaging device that enables viewing the distal end.

An endoscopic ultrasound device typically includes an ultrasound transducer on a distal tip of the instrument cord, beyond an exit aperture of the ultrasound signal channel. Signals from the ultrasound transducer may be conveyed by a suitable cable 126 back along the instrument cord to a processor 124, which can generate images in a known manner. The instrument channel may be shaped within the instrument cord to direct an instrument exiting the instrument channel through the field of view of the ultrasound system, to provide information about the location of the instrument at the target site.

The integrated flexible instrument cable exits from the distal end of the handpiece 106, and is received within the instrument channel in the instrument cord 120 of the surgical scoping device 114. The integrated flexible instrument cable has a distal assembly 118 (not drawn to scale in Fig. 1) that is shaped to pass through the instrument channel of the surgical scoping device 114 and protrude (e.g. inside the patient) at the distal end of the instrument cord 120.

The structure of the distal assembly 118 discussed below may be particularly designed for use with an endoscopic ultrasound (EUS) device, whereby the maximum outer diameter of the distal end assembly 118 is equal to or less than 1.2 mm, e.g. less than 1.0 mm and the length of the electrosurgical instrument can be equal to or greater than 1.2 m.

It is desirable to be able to control the position of at least the distal end of the instrument cord 120. The body 116 may include a control actuator that is mechanically coupled to the distal end of the instrument cord 120 by one or more control wires (not shown), which extend through the instrument cord 120. The control wires may travel within the instrument channel or within their own dedicated channels. The control actuator may be a lever or rotatable knob, or any other known catheter manipulation device. The manipulation of the instrument cord 120 may be software-assisted, e.g. using a virtual three-dimensional map assembled from computer tomography (CT) images.

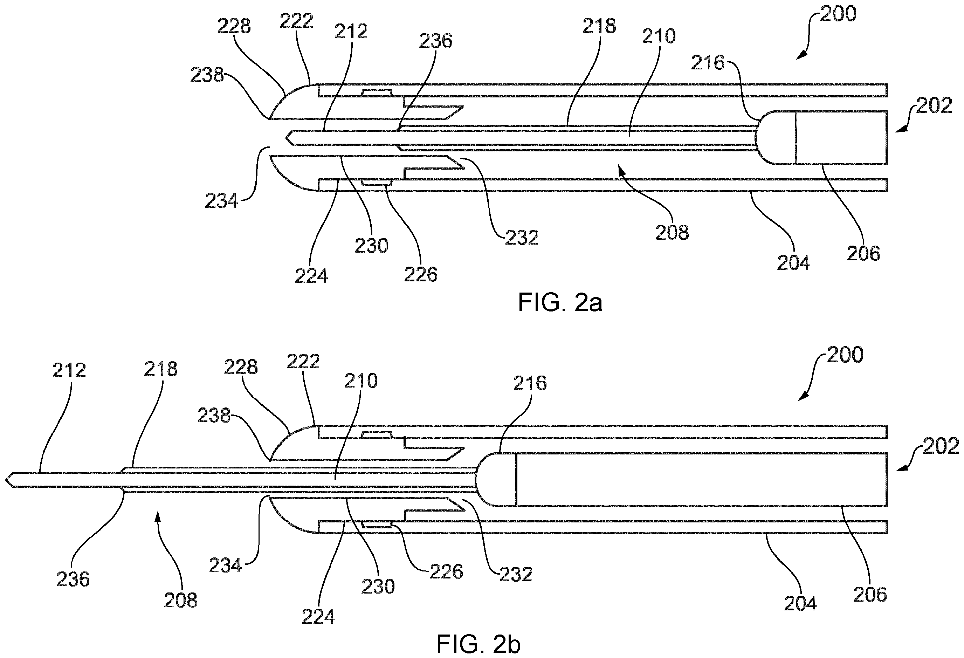

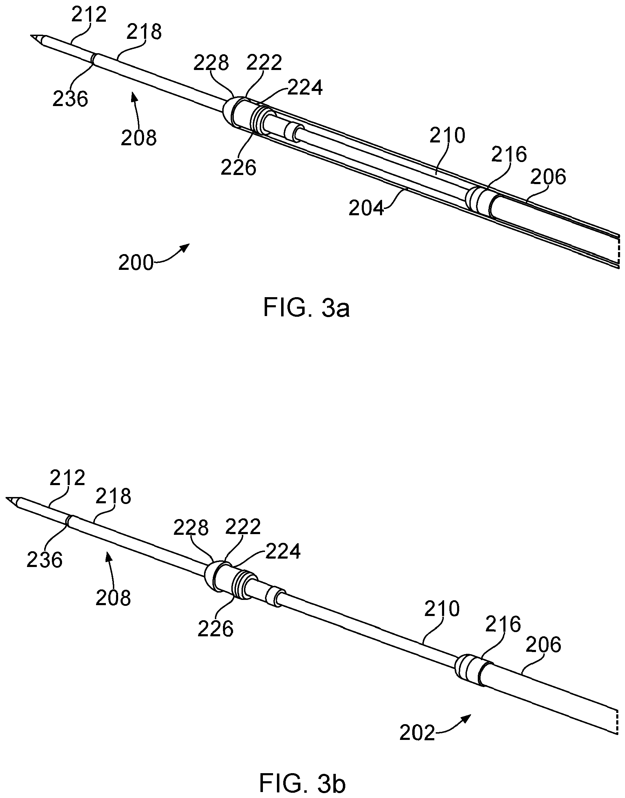

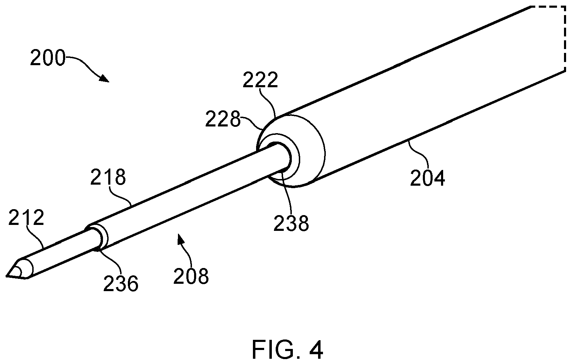

An electrosurgical instrument 200 according to an embodiment of the invention is illustrated in Figs. 2a, 2b, 3a, 3b and 4. The electrosurgical instrument 200 includes an integrated instrument cable comprising an electrosurgical device 202 disposed in a catheter 204. The catheter 204 defines a lumen in which the electrosurgical device 202 is received, and along which the electrosurgical device 202 is movable. The electrosurgical device 202 is movable along the catheter between a retracted position, which is shown in Fig. 2a, and a deployed position, which is shown in Fig. 2b. Figs. 2a and 2b show schematic cross-sectional side views of the electrosurgical instrument 200; Fig. 3a shows a perspective view of the instrument 200 where, for illustration purposes, the catheter 204 is shown as transparent to reveal the electrosurgical device 202 inside the catheter 204; and Fig. 3b shows a perspective view of the instrument 200 where, for illustration purposes, the catheter 204 has been omitted. Fig. 4 shows a perspective view of the electrosurgical instrument where the electrosurgical device 202 is in the deployed position.

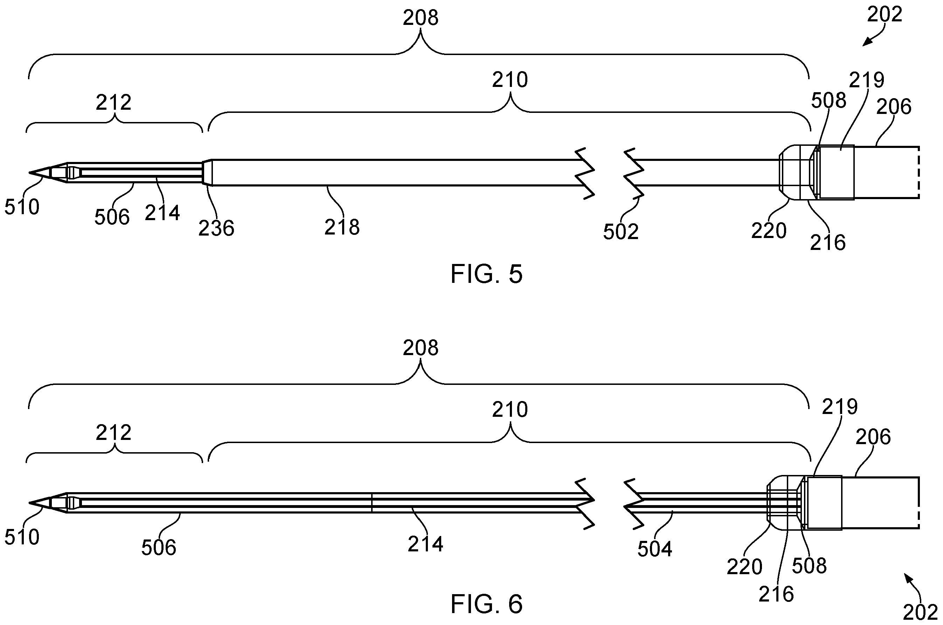

The electrosurgical device 202 is illustrated in more detail in Figs. 5 and 6. The electrosurgical device 202 includes a flexible coaxial cable 206 and a radiating tip portion 208, which is connected at a distal end of the coaxial cable 206. The coaxial cable 206 may be a conventional flexible 50 Ω coaxial cable suitable for conveying microwave and radiofrequency energy. The coaxial cable includes a centre conductor and an outer conductor that are separated by a dielectric material. The coaxial cable 206 is connectable at a proximal end to a generator, e.g. to generator 102, to receive microwave and/or radiofrequency energy.

The radiating tip portion 208 includes a proximal coaxial transmission line 210 and a distal needle tip 212 formed at a distal end of the proximal coaxial transmission line 210. The proximal coaxial transmission line 210 is electrically connected to the distal end of the coaxial cable 206 to receive the electromagnetic energy from the coaxial cable 206 and convey it to the distal needle tip 212. The distal needle tip 212 is configured to deliver the received electromagnetic energy into target biological tissue. In the example shown, the distal needle tip 212 is configured as a half wavelength transformer to deliver microwave energy into target biological tissue, to ablate the target tissue.

An inner conductor 214 of the proximal coaxial transmission line 210 is electrically connected to the centre conductor of the coaxial cable 206. The radiating tip portion 208 is secured to the coaxial cable 206 via a collar 216 mounted over a junction between the coaxial cable 206 and the radiating tip portion 208. The collar 216 is made of a conductive material (e.g. brass), and electrically connects the outer conductor of the coaxial cable 206 to an outer conductor 218 of the proximal coaxial transmission line 210. The outer conductor 218 is formed of a tube of nitinol.

The collar 216 includes a substantially cylindrical body 219 which is mounted on the distal end of the coaxial cable 206 and which is electrically connected to the outer conductor of the coaxial cable 206. The collar 216 further includes a distal portion 220 which extends from the body 219 of the collar 216 to a proximal end of the outer conductor 218 of the proximal coaxial transmission line 210. The distal portion 220 of the collar 216 includes a distal surface which is rounded. This may reduce friction between the electrosurgical device 202 and the catheter 204 when the electrosurgical device 202 is moved along the catheter 204, by avoiding sharp edges at the interface between the coaxial cable 206 and the radiating tip portion 208. This may also facilitate moving the electrosurgical device 202 along the catheter 204 when the catheter 204 is in retroflex.

The radiating tip portion 208 has a smaller outer diameter than the coaxial cable 206. This may enable the radiating tip portion 208 to be more flexible than the coaxial cable 206, which may facilitate bending of the radiating tip portion 208 and/or guiding of the radiating tip portion to an awkward treatment site. Making the outer diameter of the radiating tip portion 208 smaller may also reduce the size of an insertion hole made when the radiating tip portion 208 is inserted into tissue, which may minimise bleeding and facilitate healing. Preferably, the radiating tip portion may have an outer diameter that is 1.2 mm or less, e.g. 1.0 mm or 0.9 mm. The coaxial cable 206 may have an outer diameter that is around 2.0 mm.

The catheter 204 is a flexible tube made of an insulating material (e.g. PEEK or PTFE). The catheter 204 is dimensioned to be insertable into the working channel of a surgical scoping instrument. The catheter 204 defines a lumen in which the electrosurgical device 202 is received, and along which the electrosurgical device 202 is movable.

A plug 222 is mounted at a distal end of the catheter 204. The plug 222 has a body portion 224 which is disposed inside the distal end of the catheter 204. The body portion 224 includes a barb (or bulge) 226 disposed on an outer surface of the body portion 224, which forms an interference fit with the catheter 204, in order to secure the plug 222 at the distal end of the catheter 204. In this manner, the plug 222 may be secured at the distal end of the catheter 204 without having to use adhesive (although adhesive may be used to further secure the plug 222 to the catheter 204). A distal surface 228 of the plug 222, i.e. a surface exposed at the distal end of the catheter 204, is rounded. The rounded distal surface 228 of the plug 222 serves to avoid sharp edges around the distal end of the catheter 204. This may facilitate insertion of the electrosurgical device 200 along an instrument channel of a surgical scoping device, by reducing friction between the catheter 204 and the instrument channel at the distal end of the catheter 204. The plug may be made of PEEK or some other insulating material.

The plug 222 has a longitudinal passageway 230 defined therethrough. The passageway 230 is dimensioned to enable the radiating tip portion 208 to pass through it, but to prevent the coaxial cable 206 from passing through it. In this manner, the electrosurgical device 202 may be advanced along the catheter 204, to cause the radiating tip portion 208 to pass through the passageway so that a length of the radiating tip portion 208 protrudes beyond the distal end of the catheter 204 (e.g. as shown in Fig. 2b).

The passageway 230 includes a proximal opening 232 located at a proximal end of the body portion 224 inside the catheter 204. A distal opening 234 of the passageway 230 is located in the distal surface 228 of the plug 222. When the electrosurgical device 202 is in the deployed position (e.g. Fig. 2b), the radiating tip portion 208 protrudes through the distal opening 234 of the passageway 230. A length of the passageway 230 is greater than a length of the distal needle tip 212, i.e. a distance between the proximal opening 232 and the distal opening 234 of the passageway 230 is greater than the length of the distal needle tip 212. In this manner, as shown in Fig. 2a, when the electrosurgical device 202 is in the retracted position, the distal needle tip 212 may be entirely contained within the passageway 230 in the plug 222. In particular, a lip 236 formed by a distal end of the outer conductor 218 may be located inside the passageway 230 when the electrosurgical device 202 is in the retracted position. This may prevent the lip 236 from catching on the proximal opening 232 of the passageway 230 when the electrosurgical device 202 is moved from the retracted position to the deployed position.

The lip 236 of the outer conductor 218 is chamfered, e.g. sloped, to further prevent the lip 236 from catching on the proximal opening 232 of the passageway 230. The proximal opening 232 of the passageway is flared outwards, i.e. a diameter of the opening 232 increases in the proximal direction. In this manner, the flared proximal opening 232 acts to funnel the radiating tip portion 208 into the passageway, to avoid the radiating tip portion 208 from catching on the proximal opening 232 and to facilitate moving the radiating tip portion 208 through the passageway 230.

The passageway 230 serves to guide the radiating tip portion 208 as the electrosurgical device 202 is moved between the retracted and deployed positions. The passageway 230 is centred about a longitudinal axis of the catheter 204, such that the passageway 230 acts to centralise the radiating tip portion 208 when the radiating tip portion 208 is moved through the passageway 230.

In Fig. 2a, the electrosurgical device 202 is in the retracted position, with the distal needle tip 212 located within the passageway 230 in the plug 222. This means that the radiating tip portion 208 does not protrude from the catheter 204. In this configuration, the radiating tip portion 208 is therefore protected by the catheter 204 and the plug 222. The electrosurgical instrument 200 may be inserted into the instrument channel of a surgical scoping device and guided into position with the electrosurgical device 202 in the retracted position. This may facilitate inserting the electrosurgical instrument 200 into the instrument channel, as it prevents the radiating tip portion 208 from catching in the instrument channel.

Once the electrosurgical instrument 200 has been guided to a desired position, the electrosurgical device 202 may be moved into the deployed position. From the retracted position shown in Fig. 2a, this may be achieved by moving the electrosurgical device 202 in a distal direction along the catheter 204, to cause the radiating tip portion 208 to protrude through the distal opening 234 of the passageway 230 in the plug 222. The electrosurgical device 202 may be moved until the deployed position showed in Fig. 2b is reached. As the electrosurgical device 202 protrudes from the distal opening 234 of the passageway 230, the radiating tip portion 208 may be inserted into biological tissue. Once the distal needle tip 212 reaches a target treatment site (e.g. a tumour), microwave energy may be delivered to the distal needle tip to ablate the target tissue.

Once the target tissue has been treated, the electrosurgical device 202 may be moved back into the retracted position so that the radiating tip portion 208 is no longer exposed. This is achieved by moving the electrosurgical device 202 along the catheter 204 in a proximal direction.

A sharp lip 238 is provided around the distal opening 234 of the passageway 230. The sharp lip 238 serves to scrape any tissue or blood that is stuck on the radiating tip portion 208 when the electrosurgical device 202 is moved from the deployed position to the retracted position. This may prevent tissue and or blood from being dragged into the catheter when the radiating tip portion 208 is pulled back into the catheter 204, which could contaminate the catheter 204 or impede movement of the electrosurgical device 202 in the catheter.

We will now describe the structure of the radiating tip portion 208 of the electrosurgical device 202 in more detail, with reference to Figs. 5 and 6. For illustration purposes, the outer conductor 218 is omitted from Fig. 6, to reveal an inner structure of the radiating tip portion 208. Also for illustration purposes, a section of the proximal coaxial transmission line 210 has been omitted in Figs. 5 and 6, as indicated by broken lines 502.

The proximal coaxial transmission line 210 includes a proximal dielectric sleeve 504 which is disposed around the inner conductor 214. The outer conductor 218 is formed on an outer surface of the proximal dielectric sleeve 504. A distal dielectric sleeve 506 is disposed around a distal portion of the inner conductor 214 to form the distal needle tip 212. The distal dielectric sleeve 506 is made of a different dielectric material compared to the proximal dielectric sleeve 504. In one example, the proximal dielectric sleeve 504 may be made of PTFE (e.g. it may be a PTFE tube) and the distal dielectric sleeve may be made of PEEK. A distal portion of the outer conductor 218 overlays a proximal portion of the distal dielectric sleeve 506. In this manner, a distal portion of the proximal coaxial transmission line 210 includes the proximal portion of the distal dielectric sleeve 506. The materials of the proximal and distal dielectric sleeves and the length of the overlap between the outer conductor 518 and the distal dielectric sleeve 506 may be selected in order to adjust an electrical length of the radiating tip portion 208 to assist with impedance matching to target tissue.

The outer conductor 218 may be made of nitinol, which is flexible and provides a sufficient longitudinal rigidity to pierce tissue (e.g. the duodenum wall). The inner conductor is made of a stainless steel core that is plated with a silver coating. The stainless steel core provides additional rigidity to the radiating tip portion 208, whilst the silver coating may increase the conductivity of the inner conductor 214 to reduce losses in the radiating tip portion 208.

A dielectric spacer 508 is mounted at the junction between the radiating tip portion 208 and the coaxial cable 206. The dielectric spacer 508 is disposed inside the collar 216, and is mounted around the inner conductor 214. In this manner, the dielectric spacer 508 is disposed between the inner conductor 214 and the collar 216. This may increase a breakdown distance between the inner conductor 214 and the collar 216 at the junction between the coaxial cable 206 and the radiating tip portion 208. This may improve electrical safety of the device at the junction. The dielectric spacer 508 may, for example, be a PTFE washer or a washer made of another suitable insulating material.

As mentioned above, the distal needle tip 212 is configured as a half wavelength transformer for delivering microwave energy into tissue. Thus, when microwave energy is delivered to the distal needle tip 212, the distal needle tip 212 may radiate the microwave energy into surrounding tissue. The distal needle tip 212 includes a pointed tip 510 at its distal end, to facilitate insertion of the radiating tip portion 208 into target tissue. The pointed tip 510 is made of a dielectric material having a higher rigidity than the distal dielectric sleeve 506. This may enable the pointed tip 510 to be sharper, and it may facilitate sharpening of the pointed tip 510. For example, the pointed tip 510 may be made of Zirconia.

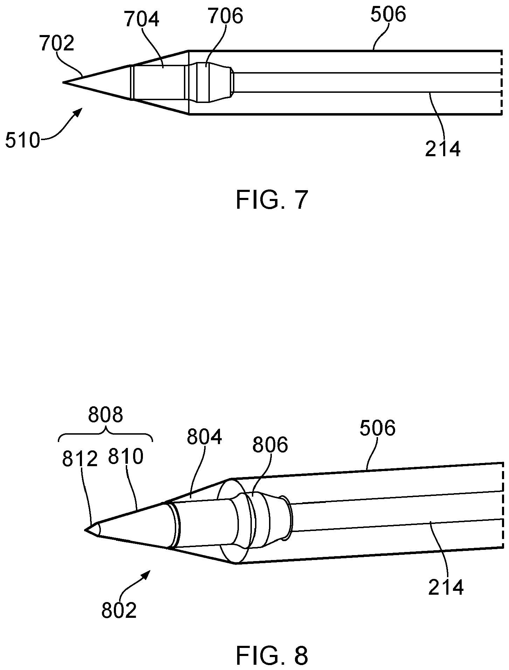

Fig. 7 shows a more detailed view of the pointed tip 510 mounted at the distal end of the distal dielectric sleeve 506. The pointed tip 510 includes a tapered portion 702 which tapers to a fine point and which serves to pierce tissue. The pointed tip also includes a body 704 which extends from a proximal end of the tapered portion 702, and which is received in a bore at the distal end of the distal dielectric sleeve. The body 704 of the pointed tip 510 includes a protrusion (or bulge) 706 which forms an interference fit with a wall of the bore, in order to hold the body 704 in the bore. In this manner, a "push-fit" connection may be formed when the body 704 is inserted into the bore. This may enable the pointed tip 510 to be mounted in the distal dielectric sleeve 506 without adhesive. This may also facilitate exchanging the pointed tip 510, e.g. if the pointed tip 510 is damaged.

Fig. 8 shows an example of a different pointed tip 802 mounted at the distal end of the distal dielectric sleeve 506. The pointed tip 802 includes a body 804 which is disposed in the bore at the distal end of the distal dielectric sleeve 506. The body 804 includes a protrusion 806 for securing the body 804 in the bore. The pointed tip 802 further includes a tapered portion 808 which protrudes from the bore in the distal dielectric sleeve 506 and tapers to a fine point for piercing tissue. The tapered portion 808 includes a proximal portion 810 which is tapered at a first angle relative to the longitudinal direction, and a distal portion 812 which is tapered at a second, larger, angle relative to longitudinal direction. In this manner, the pointed tip 802 may be said to be "double-angled". The fine point of the pointed tip 802 is formed by the distal portion 812. By making the tapering angle of the distal portion 812 larger than the tapering angle of the proximal portion 810, a length of the tapered portion 808 may be reduced. This may make the pointed tip 802 less fragile, and reduce the risk of the fine point of the pointed tip 802 breaking off.

In some embodiments (not shown), the pointed tip may be made of the same material as the distal dielectric sleeve, e.g. the pointed tip may be integrally formed with the distal dielectric sleeve. The concept using a double-angled pointed tip may be applied regardless of whether the pointed tip is formed integrally with or separately from the distal dielectric sleeve. In some embodiments (not shown), a single dielectric sleeve may be provided in place of the proximal and distal dielectric sleeves, e.g. the dielectric material in the proximal coaxial transmission line and the distal needle tip may be the same.

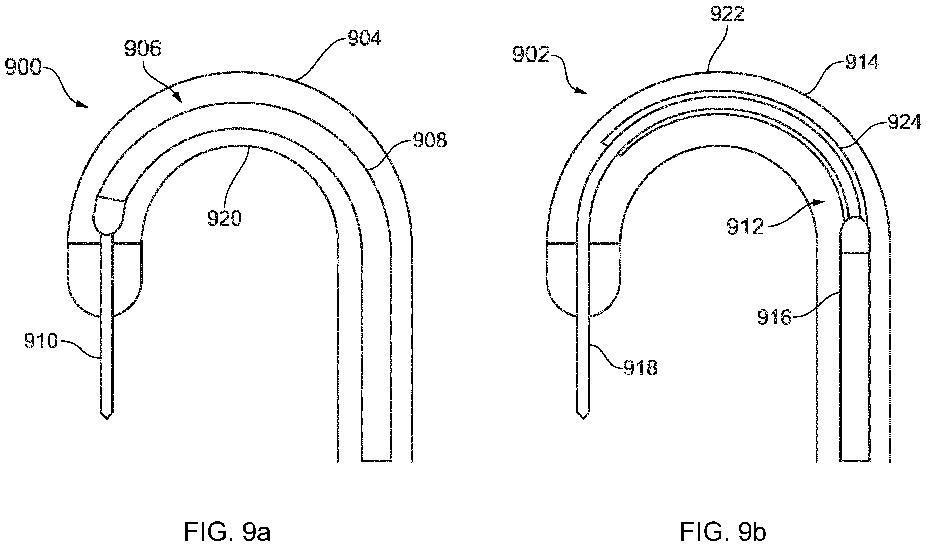

Fig. 9a shows a cross-sectional view of an electrosurgical instrument 900 according to an embodiment of the invention, where a distal portion of the instrument 900 is in retroflex. Fig. 9b shows a cross-sectional view of an electrosurgical instrument 902 according to another embodiment of the invention, where a distal portion of the instrument 902 is in retroflex.

Electrosurgical instruments 900 and 902 have a similar configuration to electrosurgical instrument 200 described above. Electrosurgical instrument 900 includes a catheter 904 in which an electrosurgical device 906 is disposed. The electrosurgical device 906 includes a coaxial cable 908 having a radiating tip portion 910 disposed at a distal end of the coaxial cable 908. The electrosurgical device 906 is movable along the catheter 904 between a retracted position where the radiating tip portion 910 is located within the catheter 904, and a deployed position where the radiating tip portion 910 protrudes from a distal end of the catheter 904. In the configuration shown in Fig. 9a, the electrosurgical device 906 is in the deployed position.

Similarly, electrosurgical instrument 902 includes an electrosurgical device 912 disposed in a catheter 914. The electrosurgical device 912 includes a coaxial cable 916 and a radiating tip portion 918 at a distal end of the coaxial cable 916. The electrosurgical device 912 is movable along the catheter 914 between a retracted position where the radiating tip portion 918 is located within the catheter 914, and a deployed position where the radiating tip portion 918 protrudes from a distal end of the catheter 914. In the configuration shown in Fig. 9b, the electrosurgical device 912 is in the deployed position.

In Fig. 9a, the distal portion 920 of the catheter 904 is in retroflex, i.e. the distal portion 920 of the catheter 904 is bent. As can be seen from Fig. 9a, a distal portion of the coaxial cable 908 is located in the distal portion 920 of the catheter 904, and is also bent. Therefore, in order to move the electrosurgical device 906 between the retracted position and the deployed position (shown in Fig. 9a), it is necessary to bend the distal portion of the coaxial cable 908. For example, when the electrosurgical device 906 is moved from the retracted position to the deployed position, the distal portion of the coaxial cable 908 must be pushed through the bent distal portion 920 of the catheter 904. When the electrosurgical device 906 is moved from the deployed position to the retracted position, the distal portion of the coaxial cable 908 must be unbent as it is pulled out of the bent distal portion 920 of the catheter 904.

Bending and unbending of the coaxial cable 908 may require a large force, due to the stiffness of the coaxial cable 908. Coaxial cables conventionally used in electrosurgical devices, such as the Sucoform 86 coaxial cable, have a relative stiff (e.g. heavily tinned) outer jacket. Whilst such an outer jacket may facilitate actuation of the device along a straight path, it may require a large force to bend the cable. As a result, when the distal portion of the catheter 904 is in retroflex, it may be necessary to apply a large force to the electrosurgical device 906 in order to move it between the retracted and deployed positions. This may result in a large amount of friction between the coaxial cable 908 and the catheter 904 in the bent portion 920, which may reduce the accuracy with which a position of the radiating tip portion 910 can be controlled.

The radiating tip portion 918 of electrosurgical device 912 (Fig. 9b) is longer than the radiating tip portion 910 of electrosurgical device 906 (Fig. 9a). As a result, when a distal portion 922 of the catheter 914 is bent (e.g. in retroflex, as shown in Fig. 9b), it may not be necessary to move the coaxial cable 916 through the bent portion 922 of the catheter 914 when the electrosurgical device 906 is moved to the deployed position. The radiating tip portion 918 may be more flexible than the coaxial cable 916, as the radiating tip portion 918 has a smaller diameter than the coaxial cable and does not have a rigid outer jacket. Thus, only a relatively small force may be required to bend the radiating tip portion 918 when it is moved through the bent distal portion 922 of the catheter 914.

As shown in Fig. 9b, by making the radiating tip portion 918 sufficiently long, the distal end of the coaxial cable 916 does not enter the bent distal portion 922 of the catheter 914 when the electrosurgical device 912 is in the deployed position, such that the distal end of the coaxial cable 916 does not need to be bent when moving the electrosurgical device 912 between the retracted and deployed positions. Preferably, the radiating tip portion may be 140 mm or longer. This may ensure that the coaxial cable 916 does not enter the retroflex portion of the catheter 914 when the electrosurgical device 912 is in the deployed position.

The configuration shown in Fig. 9b may thus reduce friction between the electrosurgical device 912 and the catheter 914 when the electrosurgical device 912 is moved along the catheter 914. This may improve control of the position of the radiating tip portion 918.

The electrosurgical device 912 further includes an outer sheath 924 disposed around a proximal portion of the radiating tip portion 918. The outer sheath 924 is made of or coated with a non-stick material, e.g. the outer sheath 924 may be a tube of PTFE. The outer sheath 924 may serve to reduce friction between the radiating tip portion 918 and the bent distal portion 922 of the catheter 914. This may facilitate moving the electrosurgical device 912 between the retracted and deployed positions. The outer sheath 924 may also serve to reduce lateral movement of the radiating tip portion 918 within the catheter, by acting as a spacer between the radiating tip portion 918 and the catheter 914. This may facilitate movement of the radiating tip portion along the bent distal portion 922 of the catheter 914.

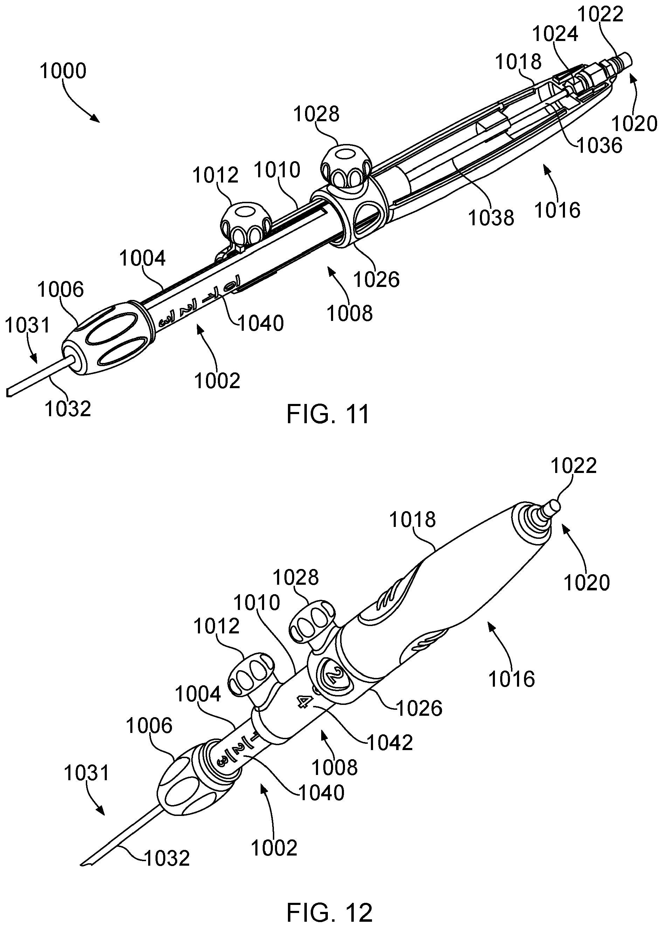

Fig. 10 shows a cross-sectional diagram of a handpiece 1000 according to an embodiment of the invention. A perspective view of the handpiece 1000 is shown in Fig. 11, where parts of components of the handpiece 1000 have been removed to reveal an internal structure of the handpiece 1000. A further perspective view of the handpiece 1000 is shown in Fig. 12. The handpiece 1000 may be used with an electrosurgical instrument of the invention, e.g. electrosurgical instrument 200, 900, 902, in order to move the electrosurgical device of the instrument along the catheter of the instrument, between the retracted position and the deployed position.

The handpiece 1000 includes a first section 1002 which has a generally cylindrical hollow body 1004. A connector 1006 is provided at a distal end of the hollow body 1004, the connector 1006 being adapted to mount the handpiece onto an input port of an instrument channel of a surgical scoping device. The connector 1006 may be configured to mate with a corresponding connector on the input port. The connector 1006 may include a luer fitting (or some other suitable fitting), to provide a leak-free connection between the handpiece 1000 and the instrument channel of the surgical scoping device.

The handpiece 1000 further includes a second section 1008. The second section 1008 is formed of a generally cylindrical hollow body 1010 which is telescopically mounted on the first section 1002, such that the second section 1008 is longitudinally slidable over a length of the first section 1002. The second section 1008 includes a fixing screw 1012 for fixing the position of the second section 1010 relative to the first section 1002. The fixing screw 1012 is engaged in a threaded hole in a sidewall of the hollow body 1010. Tightening the fixing screw 1012 in the threaded hole causes the fixing screw 1012 to engage the body 1004 of the first section 1002 to fix the position of the second section 1010 relative to the first section 1002. Loosening the fixing screw 1012 in the threaded hole disengages the fixing screw from the first section 1002, so that the second section 1008 can be slid relative to the first section 1002.

The second section 1008 includes a holder 1014 disposed on an inside of the body 1010 of the second section 1008. The holder 1014 is configured to hold a proximal end of a catheter of an electrosurgical instrument. The holder 1014 may, for example, be a clip or a clamp arranged to hold the proximal end of the catheter. In another example, the holder 1014 may be a surface to which the proximal end of the catheter may be secured (e.g. using an adhesive). In this manner, when a proximal end of a catheter is held in the holder 1014, a position of the catheter is fixed relative to the second section 1008. Thus, sliding the second section 1008 relative to the first section 1002 causes the catheter to move relative to the first section 1002.

The handpiece 1000 further includes a third section 1016. The third section 1016 is formed of a generally cylindrical hollow body 1018 which is telescopically mounted on the second section 1008, such that the third section 1016 is longitudinally slidable over a length of the second section 1008. A coaxial connector 1020 is mounted on a proximal end 1019 of the body 1018. A proximal end 1022 of the coaxial connector 1020 is exposed on an outside of the body 1018 of the third section 1016. The proximal end 1022 of the coaxial connector 1020 is connectable to an electrosurgical generator, e.g. via a connecting cable (not shown).

A distal end 1024 of the coaxial connector 1020 is disposed inside the hollow body 1018 of the third section 1016. The distal end 1024 of the coaxial connector 1020 is arranged to receive the proximal end of a coaxial cable of an electrosurgical device. For example, the distal end 1024 of the coaxial connector 1020 may include an inner conductor and an outer conductor which can, respectively, be electrically connected to a centre conductor and an outer conductor of the coaxial cable (e.g. via soldered or welded connections). Alternatively, the distal end 1024 of the coaxial connector 1020 may be adapted to mate with a corresponding connector on the proximal end of the coaxial cable. The coaxial connector 1020 is arranged to convey electromagnetic energy from a cable connected to its proximal end 1022 to a coaxial cable connected to its distal end 1024.

A slidable limiter 1026 is mounted on an outer surface of the body 1010 of the second section 1008. The slidable limiter 1026 is slidable along the outer surface of the body 1010 of the second section 1008. The position of the slidable limiter 1026 can be fixed relative to the second section 1008 via a fixing screw 1028 which is engaged in a threaded hole in the slidable limiter. The slidable limiter 1026 includes a stopping surface 1030 which is arranged to abut against a distal surface 1029 of the body 1018 of the third section 1016 when the third section is moved in the distal direction (i.e. towards the first section 1002), to prevent further motion of the third section 1016 in the distal direction. In this manner, a range of motion of the third section 1016 relative to the second section 1008 may be adjusted by adjusting the position of the slidable limiter 1026 on the second section 1008.

In the examples shown in Figs. 10 and 11, a proximal end of an electrosurgical instrument 1031 is mounted in the handpiece 1000. The electrosurgical instrument may be an electrosurgical instrument according to an embodiment of the invention, e.g. as described above. A proximal end of a catheter 1032 of the instrument 1031 is held in the holder 1014 in the second section 1016 of the handpiece 1000. A proximal end of a coaxial cable 1034 of the instrument 1031 is electrically connected to the distal end 1024 of the coaxial connector 1020 in the third section 1016 of the handpiece 1000. The coaxial cable 1034 is disposed in the catheter 1032 and is slidable along the catheter 1032. The electrosurgical instrument 1031 extends within the hollow bodies of the first, second and third sections of the handpiece 1000. A distal portion of the electrosurgical instrument 1031 exits from the handpiece through the connector 1006 at the distal end of the first section 1002.

A reinforcing tube 1036 is provided around an outer surface of a proximal portion of the coaxial cable 1034. The reinforcing tube 1036 is secured to the coaxial connector 1020. The reinforcing tube 1036 is made of a rigid material, e.g. a rigid metal or plastic. The reinforcing tube 1036 serves to increase the longitudinal rigidity of proximal portion of the coaxial cable 1034, to facilitate transmission of longitudinal force to the coaxial cable 1034 when the third section 1016 is moved relative to the second section 1008. A proximal portion 1038 of the catheter 1032 has an expanded diameter to enable the proximal portion of the coaxial cable 1034 and the reinforcing tube 1036 to slide within the catheter 1032.

In use, the distal portion of the electrosurgical instrument 1031 that exits from the connector 1006 may be inserted into the instrument channel of a surgical scoping device. Then, the connector 1006 of the handpiece 1000 may be connected to a corresponding connector on an input port of the surgical scoping device to secure the handpiece 1000 to the surgical scoping device. Subsequently, a length of the catheter 1032 in the instrument channel may be adjusted by sliding the second section 1008 relative to the first section 1002. As the catheter 1032 is fixed relative to the second section 1008 (via the holder 1014), moving the second section 1008 relative to the first section 1002 changes the length of the catheter 1032 which exits from the connector 1006, and hence the length of the catheter 1032 located in the instrument channel. A set of markers 1040 is provided on an outer surface of the body 1004 of the first section 1002. Each marker of the set of markers 1040 indicates a length of the catheter exiting from the handpiece when a distal surface 1041 of the second section 1008 is aligned with that marker. Thus, the second section 1008 may be moved along the first section 1002 to a marker on the first section 1002 corresponding to a desired length of the catheter 1032 in the instrument channel. When the desired length is obtained, the position of the second section 1008 may be fixed relative to the first section 1002, by tightening the fixing screw 1012.

After adjusting the length of the catheter 1032, the coaxial cable 1034 may be moved along the catheter 1032, e.g. to expose or retract the radiating tip portion of the instrument 1031. The coaxial cable 1034 may be moved along the catheter 1032 by sliding the third section 1016 relative to the second section 1008. Longitudinal motion of the third section 1016 relative to the second section 1008 is transmitted to the distal end of the coaxial cable 1034 which is connected to the coaxial connector 1020 in the third section 1016. The reinforcing tube 1036 prevents the proximal end of the coaxial cable 1034 from bending, so that longitudinal motion of the third section 1016 is transmitted to the coaxial cable 1034. When the third section 1016 is moved in a proximal direction relative to the second section 1008, the reinforcing tube slides into the expanded proximal portion 1038 of the catheter 1032.

As the catheter 1032 is fixed relative to the second section 1008, sliding the third section 1016 relative to the second section 1008 causes the coaxial cable 1034 to slide within the catheter 1032. Thus, when the handpiece 1000 is used with an electrosurgical instrument of the invention (e.g. electrosurgical instrument 200), the electrosurgical device may be moved between the retracted and deployed positions by moving the third section 1016 backwards and forwards relative to the second section 1008. Specifically, the electrosurgical device may be moved to the deployed position by moving the third section 1016 in a proximal direction relative to the second section 1008, and the electrosurgical device may be moved to the retracted position by moving the third section 1016 in a distal direction relative to the second section 1008.

A user may adjust the position of the slidable limiter 1026 on the second section 1008 to set a maximum forward position of the third section 1016 relative to the second section 1008. This may set a maximum extension of the radiating tip portion beyond the distal end of the catheter 1032 when the electrosurgical device is in the deployed position. In this manner, the slidable limiter 1026 may prevent the radiating tip portion from being pushed too far beyond the distal end of the catheter 1032. A set of markers 1042 is provided on an outer surface of the body 1010 of the second section 1008. Each marker of the set of markers 1042 indicates a maximum extension of the radiating tip portion beyond the distal end of the catheter 1032 when the slidable limiter 1026 is aligned with that marker and the distal surface 1029 of the third section 1016 abuts against the stopping surface 1030 of the slidable limiter 1026. The slidable limiter 1026 may be thus be moved to a corresponding marker of the set of markers 1042 to set a desired maximum extension of the radiating tip portion.

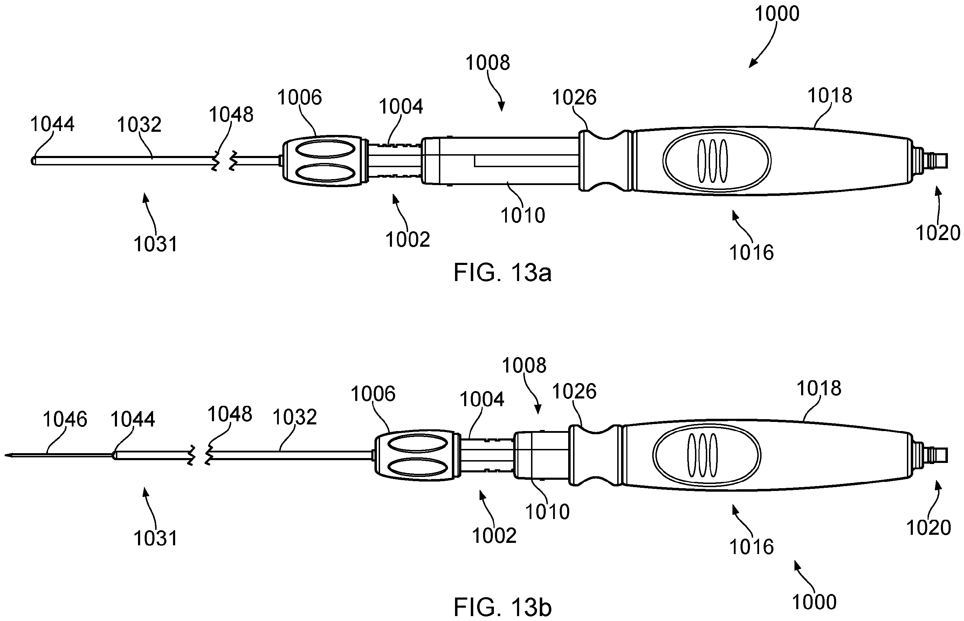

Figs. 13a and 13b illustrate an operation of the handpiece 1000 to move an electrosurgical device of instrument 1031 between a retracted position and a deployed position. In Fig. 13a, the electrosurgical device of instrument 1031 is in a retracted position, i.e. a radiating tip portion of the electrosurgical device does not protrude beyond a distal end 1044 of the catheter 1032. In Fig. 13b, the electrosurgical device of instrument 1031 is in a deployed position, i.e. a radiating tip portion 1046 of the electrosurgical device protrudes beyond the distal end 1044 of the catheter 1032. In this configuration, electromagnetic energy may be delivered to the radiating tip portion 1046 via the coaxial connector 1020 to deliver the energy into target tissue.

To go from the configuration shown in Fig. 13a to that shown in Fig. 13b, first, the limiter 1026 may be moved to a desired position on the body 1010 of the second section 1008. Then, the third section 1016 may be slid over the second section 1008 in the distal direction. The third section 1016 may be advanced until the distal surface 1029 of the third section 1016 abuts against the slidable limiter 1026, as shown in Fig. 13b. Once treatment of the target tissue has been completed, the radiating tip portion 1046 may be withdrawn back into the catheter 1032 by sliding the third section 1016 in the proximal direction relative to the second section, to return to the configuration shown in Fig. 13a. For illustration purposes, a length of the instrument 1031 is omitted from Figs. 13a and 13b, as indicated by dashed lines 1048.

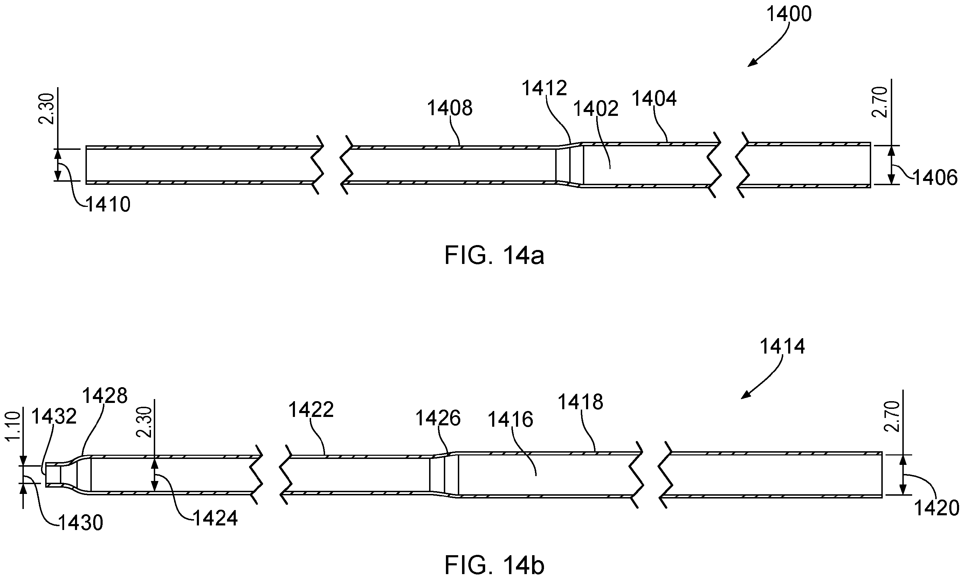

Fig. 14a shows cross-sectional view of a catheter 1400 that may be used as part of an electrosurgical instrument of the invention. The catheter 1400 defines a lumen 1402 extending therethrough and in which an electrosurgical device is receivable. The catheter 1400 includes a proximal section 1404 having a first diameter 1406 and a distal section 1408 having a second, smaller, diameter 1410. Example dimensions for the first and second diameters are 2.70 mm and 2.30 mm, respectively. A stepped section 1412 links the proximal section 1404 and the distal section 1408. The larger diameter of the proximal section 1404 is arranged so that the proximal section 1404 can receive a proximal section of a coaxial cable of an electrosurgical device which includes a reinforcing element (which increases an effective outer diameter of the proximal section of the coaxial cable).

Fig. 14b shows a cross-sectional view of another catheter 1414 that may be used as part of an electrosurgical instrument of the invention. The catheter 1414 defines a lumen 1416 extending therethrough and in which an electrosurgical device is receivable. The catheter 1414 includes a proximal section 1418 having a first diameter 1420 and an intermediate section 1422 having a second, smaller, diameter 1424. The larger diameter of the proximal section 1404 is arranged so that the proximal section 1404 can receive a proximal section of a coaxial cable of an electrosurgical device which includes a reinforcing element. The proximal section 1418 and intermediate section 1422 are joined by a stepped section 1426.

A tapered section 1428 is located at a distal end of the catheter 1414. The tapered section 1428 serves to reduce a diameter of the catheter 1414 at the distal end of the catheter from the second diameter 1424 to a third, smaller, diameter 1430. The tapered section 1428 defines a distal opening 1432 of the catheter 1414, a diameter of the distal opening 1432 being the third diameter 1430. The third diameter 1432 is set so that it is larger than an outer diameter of a radiating tip portion of the electrosurgical instrument, and smaller than an outer diameter of a coaxial cable of the instrument. In this manner, the radiating tip portion may protrude through the distal opening 1432 (e.g. when the electrosurgical device is in a deployed position), but the coaxial cable (which has a larger outer diameter than the radiating tip portion) may be prevented from passing through the distal opening 1432. In this manner, the tapered section 1428 may fulfil a similar function to the plug 222 at the end of catheter 204. By providing a tapered section 1428 at the distal end of the catheter 1414, it may thus not be necessary to provide a plug at the distal end of the catheter 1414. This may simplify construction of the electrosurgical instrument. Example dimensions for the first, second and third diameters are 2.70 mm, 2.30 mm and 1.10 mm respectively.

The different sections of catheters 1400 and 1414 may be made by bonding tubes of different diameters together. Alternatively, catheters 1400 and 1414 may be made from a single tube that is formed by a multi-diameter extrusion process. This may be done by extruding a tube at a first diameter, and then post processing a portion of the tube to remould that portion to a different (e.g. larger) diameter.

## Claims

1. A handpiece (1000) for controlling movement of an electrosurgical device along a lumen of a catheter, the handpiece comprising:
a first section (1002) having a connector (1006) for connecting a distal end of the handpiece to an instrument port of a surgical scoping device; and
a second section (1008) connected to the first section and movable along a length of the first section; and
a third section (1016) connected to the second section and movable along a length of the second section;
**characterised in that**:
the second section has a holder (1014) for holding a proximal end of the catheter, whereby relative movement between the second section and first section is arranged to control a length of catheter that extends out of a distal end of the handpiece; and
the third section has a coaxial connector (1020) arranged to receive a proximal end of a coaxial cable that is conveyed within the lumen of the catheter, whereby relative movement between the third section and second section is arranged to control a relative position of the coaxial cable within the catheter.

2. A handpiece according to claim 1, wherein the second section has a fixing mechanism (1012) for fixing a position of the second section relative to the first section.

3. A handpiece according to claim 1 or 2, wherein the second section includes a limiter (1026) that is movable along a length of the second section, and wherein the limiter is arranged to restrict motion of the third section relative to the second section.

4. A handpiece according to any preceding claim,
wherein the first section and the second section are telescopically arranged, the second section being slidable along the length of the first section.

5. A handpiece according to any preceding claim,
wherein the second section and the third section are telescopically arranged, the third section being slidable along the length of the second section.

6. An electrosurgical system (100) for treating biological tissue, the system comprising:
an electrosurgical generator (102) arranged to supply microwave energy;
a surgical scoping device (114) having a flexible insertion cord (120) for insertion into a patient's body, wherein the flexible insertion cord has an instrument channel running along its length;
an electrosurgical instrument (200) comprising:
a flexible catheter (204) having a lumen extending therethrough, the catheter being dimensioned to be insertable through the instrument channel of the surgical scoping device; and
an electrosurgical device (202) disposed within the lumen, the electrosurgical device comprising:
a flexible coaxial cable (206) configured to convey microwave energy; and
a radiating tip portion (208) connected at a distal end of the coaxial cable to receive the microwave energy, the radiating tip portion having a smaller outer diameter than the flexible coaxial cable, wherein the radiating tip portion comprises:
a proximal coaxial transmission line (210) for conveying the microwave energy; and
a distal needle tip (212) at a distal end of the proximal coaxial transmission line, the distal needle tip being configured to radiate the microwave energy into biological tissue,
wherein the electrosurgical device is longitudinally movable within the lumen between a deployed position in which the distal needle tip protrudes beyond a distal end of the catheter, and a retracted position in which the distal needle tip portion is contained within the catheter;
wherein the electrosurgical instrument is dimensioned to fit within the instrument channel; and
a handpiece (1000) according to any one of claims 1 to 5, wherein a proximal end of the catheter of the electrosurgical instrument is held in the holder, a proximal end of the coaxial cable of the electrosurgical instrument is received in the coaxial connector, and the coaxial connector is connected to the electrosurgical generator to receive the microwave energy.

7. An electrosurgical system according to claim 6,
wherein the distal needle tip is configured to operate as a half wavelength transformer to deliver the microwave energy from the distal needle tip into biological tissue.

8. An electrosurgical system according to claim 6 or 7, wherein the catheter includes a constricted passageway (230) at its distal end, the constricted passageway being dimensioned to permit passage of the radiating tip portion and to prohibit passage of the flexible coaxial cable.

9. An electrosurgical system according to claim 8,
wherein the constricted passageway extends through a plug (222) mounted at the distal end of the catheter.

10. An electrosurgical system according to one of claims 6 to 9, wherein a distal surface (228) of the catheter is rounded.

11. An electrosurgical system according to any one of claims 8 to 10, wherein the distal needle tip is retained in the constricted passageway when in the retracted position.

12. An electrosurgical system according to one of claims 6 to 11, wherein the distal needle tip comprises a pointed tip (510) at its distal end.

13. An electrosurgical system according to claim 12,
wherein the pointed tip is made of a rigid insulating material.

14. An electrosurgical system according to claim 12 or 13, wherein distal needle tip comprises a distal dielectric sleeve (506) around a central conductive element (214), and wherein the pointed tip is secured in a bore at a distal end of the distal dielectric sleeve.

15. An electrosurgical system according to any one of clauses 12 to 14, wherein the pointed tip is made of zirconia.

## Patentansprüche

1. Handstück (1000) zur Steuerung der Bewegung einer elektrochirurgischen Vorrichtung entlang eines Lumens eines Katheters, wobei das Handstück Folgendes umfasst:
einen ersten Abschnitt (1002) mit einem Verbinder (1006) zum Verbinden eines distalen Endes des Handstücks mit einem Instrumentenanschluss einer chirurgischen Sondiervorrichtung;
und einen zweiten Abschnitt (1008), der mit dem ersten Abschnitt verbunden und entlang einer Länge des ersten Abschnitts bewegbar ist; und
einen dritten Abschnitt (1016), der mit dem zweiten Abschnitt verbunden und entlang einer Länge des zweiten Abschnitts bewegbar ist;
**dadurch gekennzeichnet, dass**:
der zweite Abschnitt eine Halterung (1014) zum Halten eines proximalen Endes des Katheters umfasst, wobei eine Relativbewegung zwischen dem zweiten Abschnitt und dem ersten Abschnitt ausgelegt ist, um die Länge des Katheters zu steuern, die sich aus einem distalen Ende des Handstücks heraus erstreckt; und
der dritte Abschnitt einen koaxialen Verbinder (1020) aufweist, der angeordnet ist, um ein proximales Ende eines Koaxialkabels aufzunehmen, das im Lumen des Katheters befördert wird, wobei die Relativbewegung zwischen dem dritten Abschnitt und dem zweiten Abschnitt ausgelegt ist, um eine relative Position des Koaxialkabels im Katheter zu steuern.

2. Handstück nach Anspruch 1, wobei der zweite Abschnitt einen Fixiermechanismus (1012) zum Fixieren einer Position des zweiten Abschnitts relativ zum ersten Abschnitt umfasst.

3. Handstück nach Anspruch 1 oder 2, wobei der zweite Abschnitt einen Begrenzer (1026) umfasst, der entlang einer Länge des zweiten Abschnitts bewegbar ist, und wobei der Begrenzer angeordnet ist, um die Bewegung des dritten Abschnitts relativ zum zweiten Abschnitt einzuschränken.

4. Handstück nach einem der vorangegangenen Ansprüche, wobei der erste Abschnitt und der zweite Abschnitt teleskopartig angeordnet sind und der zweite Abschnitt entlang der Länge des ersten Abschnitts verschiebbar ist.

5. Handstück nach einem der vorangegangenen Ansprüche, wobei der zweite Abschnitt und der dritte Abschnitt teleskopartig angeordnet sind und der dritte Abschnitt entlang der Länge des zweiten Abschnitts verschiebbar ist.

6. Elektrochirurgisches System (100) zur Behandlung von biologischem Gewebe, wobei das System Folgendes umfasst:
einen elektrochirurgischen Generator (102), der angeordnet ist, um Mikrowellenergie bereitzustellen;
eine chirurgische Sondiervorrichtung (114) mit einem flexiblen Einführkabel (120) zum Einführen in den Körper eines Patienten, wobei das flexible Einführkabel einen Instrumentenkanal aufweist, der entlang seiner Länge verläuft;
ein elektrochirurgisches Instrument (200), das Folgendes umfasst:
einen flexiblen Katheter (204) mit einem Lumen, das sich durch diesen hindurch erstreckt, wobei der Katheter dimensioniert ist, um durch den Instrumentenkanal der chirurgischen Sondiervorrichtung einführbar zu sein; und
eine elektrochirurgische Vorrichtung (202), die im Lumen angeordnet ist, wobei die elektrochirurgische Vorrichtung Folgendes umfasst:
ein flexibles Koaxialkabel (206), das ausgelegt ist, um Mikrowellenenergie zu übertragen; und
einen abstrahlenden Spitzenteil (208), der an einem distalen Ende des koaxialen Kabels verbunden ist, um die Mikrowellenenergie zu empfangen, wobei der abstrahlende Spitzenteil einen kleineren Außendurchmesser aufweist als das flexible Koaxialkabel, wobei der abstrahlende Spitzenteil Folgendes umfasst:
eine proximale Koaxial-Übertragungsleitung (210) zum Transportieren der Mikrowellenenergie; und
eine distale Nadelspitze (212) an einem distalen Ende der proximalen Koaxial-Übertragungsleitung, wobei die distale Nadelspitze ausgelegt ist, um die Mikrowellenenergie in das biologische Gewebe abzustrahlen,
wobei die elektrochirurgische Vorrichtung in Längsrichtung im Lumen zwischen einer ausgefahrenen Position, in der die distale Nadelspitze über ein distales Ende des Katheters hinausragt, und einer eingefahrenen Position bewegbar ist, in der der distale Nadelspitzenabschnitt im Katheter enthalten ist;
wobei das elektrochirurgische Instrument dimensioniert ist, um in den Instrumentenkanal zu passen; und
ein Handstück (1000) nach einem der Ansprüche 1 bis 5, wobei ein proximales Ende des Katheters des elektrochirurgischen Instruments in der Halterung gehalten wird, ein proximales Ende des Koaxialkabels des elektrochirurgischen Instruments im koaxialen Verbinder aufgenommen ist und der koaxiale Verbinder mit dem elektrochirurgischen Generator verbunden ist, um die Mikrowellenenergie zu empfangen.

7. Elektrochirurgisches System nach Anspruch 6, wobei die distale Nadelspitze ausgelegt ist, um als Halbwellenlängentransformator zu arbeiten, um die Mikrowellenenergie von der distalen Nadelspitze in biologisches Gewebe zuzuführen.

8. Elektrochirurgisches System nach Anspruch 6 oder 7, wobei der Katheter einen verengten Durchgang (230) an seinem distalen Ende umfasst, wobei der verengte Durchgang dimensioniert ist, um den Durchtritt des abstrahlenden Spitzenabschnitts zu ermöglichen und den Durchtritt des flexiblen Koaxialkabels zu verhindern.

9. Elektrochirurgisches System nach Anspruch 8, wobei sich der verengte Durchgang durch einen Stopfen (222) erstreckt, der am distalen Ende des Katheters befestigt ist.

10. Elektrochirurgisches System nach einem der Ansprüche 6 bis 9, wobei eine distale Oberfläche (228) des Katheters abgerundet ist.

11. Elektrochirurgisches System nach einem der Ansprüche 8 bis 10, wobei die distale Nadelspitze im verengten Durchgang rückgehalten ist, wenn sie in der eingefahrenen Position ist.

12. Elektrochirurgisches System nach einem der Ansprüche 6 bis 11, wobei die distale Nadelspitze eine spitz zulaufende Spitze (510) am distalen Ende aufweist.

13. Elektrochirurgisches System nach Anspruch 12, wobei die spitz zulaufende Spitze aus einem starren Isoliermaterial besteht.

14. Elektrochirurgisches System nach Anspruch 12 oder 13, wobei die distale Nadelspitze eine distale dielektrische Hülse (506) um ein zentrales leitendes Element (214) umfasst und wobei die spitz zulaufende Spitze in einer Bohrung an einem distalen Ende der dielektrischen Hülse festgelegt ist.

15. Elektrochirurgisches System nach einem der Ansprüche 12 bis 14, wobei die spitz zulaufende Spitze aus Zirkoniumoxid besteht.

## Revendications

1. Pièce à main (1000) pour commander le déplacement d'un dispositif électrochirurgical le long d'une lumière d'un cathéter, la pièce à main comprenant :
une première section (1002) présentant un connecteur (1006) pour connecter une extrémité distale de la pièce à main à un port d'instrument d'un dispositif d'endoscopie chirurgicale ; et
une deuxième section (1008) connectée à la première section et mobile le long d'une longueur de la première section ; et
une troisième section (1016) connectée à la deuxième section et mobile le long d'une longueur de la deuxième section ;
**caractérisé en ce que** :
la deuxième section présente un support (1014) pour supporter une extrémité proximale du cathéter, moyennant quoi un déplacement relatif entre la deuxième section et la première section est agencé pour commander une longueur de cathéter qui s'étend à l'extérieur d'une extrémité distale de la pièce à main ; et
la troisième section présente un connecteur coaxial (1020) agencé pour recevoir une extrémité proximale d'un câble coaxial qui est transporté dans la lumière du cathéter, moyennant quoi un déplacement relatif entre la troisième section et la deuxième section est agencé pour commander une position relative du câble coaxial à l'intérieur du cathéter.

2. Pièce à main selon la revendication 1, dans laquelle la deuxième section présente un mécanisme de fixation (1012) pour fixer une position de la deuxième section par rapport à la première section.

3. Pièce à main selon la revendication 1 ou 2, dans lequel la deuxième section comprend un limiteur (1026) qui est mobile le long d'une longueur de la deuxième section, et dans lequel le limiteur est agencé pour restreindre le mouvement de la troisième section par rapport à la deuxième section.

4. Pièce à main selon l'une quelconque des revendications précédentes, dans laquelle la première section et la deuxième section sont agencées de manière télescopique, la deuxième section pouvant coulisser le long de la longueur de la première section.

5. Pièce à main selon l'une quelconque des revendications précédentes, dans laquelle la deuxième section et la troisième section sont agencées de manière télescopique, la deuxième section pouvant coulisser le long de la longueur de la première section.

6. Système électrochirurgical (100) pour traiter des tissus biologiques, le système comprenant :
un générateur électrochirurgical (102) agencé pour fournir de l'énergie hyperfréquence ;
un dispositif d'endoscopie chirurgicale (114) présentant un cordon d'insertion flexible (120) pour une insertion dans le corps d'un patient, dans lequel le cordon d'insertion flexible présente un canal d'instrument s'étendant sur toute sa longueur ;
un instrument électrochirurgical (200), comprenant :
un cathéter flexible (204) présentant une lumière s'étendant à travers celui-ci, le cathéter étant dimensionné pour pouvoir être inséré à travers le canal d'instrument du dispositif d'endoscopie chirurgicale ; et
un dispositif électrochirurgical (202) disposé dans la lumière, le dispositif électrochirurgical comprenant :
un câble coaxial flexible (206) configuré pour transporter de l'énergie hyperfréquence ; et
une partie de pointe rayonnante (208) connectée à une extrémité distale du câble coaxial pour recevoir l'énergie hyperfréquence, la partie de pointe rayonnante présentant un diamètre extérieur plus petit que le câble coaxial flexible, dans lequel la partie de pointe rayonnante comprend :
une ligne de transmission coaxiale proximale (210) pour transporter l'énergie hyperfréquence ; et
une pointe d'aiguille distale (212) au niveau d'une extrémité distale de la ligne de transmission coaxiale proximale, la pointe d'aiguille distale étant configurée pour rayonner l'énergie hyperfréquence dans du tissu biologique,
dans lequel le dispositif électrochirurgical est mobile longitudinalement dans la lumière entre une position déployée dans laquelle la pointe d'aiguille distale fait saillie au-delà d'une extrémité distale du cathéter, et une position rétractée dans laquelle la partie de pointe d'aiguille distale est contenue à l'intérieur du cathéter ;
dans lequel l'instrument électrochirurgical est dimensionné pour s'ajuster à l'intérieur du canal d'instrument ; et
une pièce à main (1000) selon l'une quelconque des revendications 1 à 5, dans lequel une extrémité proximale du cathéter de l'instrument électrochirurgical est supportée dans le support, une extrémité proximale du câble coaxial de l'instrument électrochirurgical est reçue dans le connecteur coaxial, et le connecteur coaxial est connecté au générateur électrochirurgical pour recevoir l'énergie hyperfréquence.

7. Système électrochirurgical selon la revendication 6, dans lequel la pointe d'aiguille distale est configurée pour fonctionner comme un transformateur de demi-longueur d'onde pour délivrer l'énergie hyperfréquence à partir depuis la pointe d'aiguille distale jusque dans le tissu biologique.

8. Système électrochirurgical selon la revendication 6 ou 7, dans lequel le cathéter inclut un passage rétréci (230) au niveau de son extrémité distale, le passage rétréci étant dimensionné pour permettre le passage de la partie de pointe rayonnante et pour empêcher le passage du câble coaxial flexible.

9. Système électrochirurgical selon la revendication 8, dans lequel le passage rétréci s'étend à travers un bouchon (222) monté au niveau de l'extrémité distale du cathéter.

10. Système électrochirurgical selon l'une quelconque des revendications 6 à 9, dans lequel une surface distale (228) du cathéter est arrondie.

11. Système électrochirurgical selon l'une quelconque des revendications 8 à 10, dans lequel la pointe d'aiguille distale est retenue dans le passage rétréci lorsqu'elle est dans la position rétractée.

12. Système électrochirurgical selon l'une quelconque des revendications 6 à 11, dans lequel la pointe d'aiguille distale comprend une pointe pointue (510) au niveau de son extrémité distale.

13. Système électrochirurgical selon la revendication 12, dans lequel la pointe pointue est réalisé en un matériau isolant rigide.

14. Système électrochirurgical selon la revendication 12 ou 13, dans lequel la pointe d'aiguille distale comprend un manchon diélectrique distal (506) autour d'un élément conducteur central (214), et dans lequel la pointe pointue est fixée dans un alésage au niveau d'une extrémité distale du manchon diélectrique distal.

15. Système électrochirurgical selon l'une quelconque des revendications 12 à 14, dans lequel la pointe pointue est réalisée en zircone.
